# EUROPEAN PATENT APPLICATION

(11) **EP 4 400 594 A1**
(43) Date of publication of application: **17.07.2024**
(21) Application number: 22863668.4
(22) Date of filing: 06.09.2022
(51) Int. Cl.: C12N 15/82, C12N 15/62, A01H 5/00

(54) **IMPROVED GUIDED EDITING SYSTEM**

(30) Priority: 06.09.2021 CN 202111039979
(71) Applicant: Suzhou Qi Biodesign biotechnology Company Limited, Jiangsu 215127 (CN)
(72) Inventor: GAO, Caixia, Beijing 100101 (CN); ZONG, Yuan, Beijing 100101 (CN)
(74) Representative: Michalski Hüttermann & Partner Patentanwälte mbB
(86) International application number: PCT/CN2022/117258
(87) International publication number: WO 2023/030534

(57) **Abstract**

The present invention relates to an improved primer editing system, and a method for performing gene editing with the system.

## Description

### Technical Field

The present invention relates to the field of genetic engineering. Specifically, the present invention relates to an improved prime editing system and a method for gene editing with the prime editing system.

### Background

Many important agronomic traits depend on sequences in the genome. By directionally changing specific sequences of the genome, new heritable traits may be conferred to organisms, providing possibility for disease treatment and breeding improvement. Currently, editing on a specific sequence can be achieved by the genome editing techniques (e.g., a CRISPR/Cas technique), so that a repairing pathway of cells is activated to repair injury site. A prime editing (PE) system is a system based on the CRISPR/Cas technology that may accurately modify a target site sequence. The system is comprised of two moieties: 1) a fusion protein comprising a Cas9 nuclease having non-target strand nicking activity (Cas9-H840A) and a reverse transcriptase (RT), and 2) a pegRNA (prime editing gRNA) with a repair template (RT template) and a primer binding site (PBS) of a free single strand at the 3' end. The working principle of the system is that, the PBS binds to the free single strand generated by Cas9-H840A, to guide the fusion protein to bind to a designated site, a single-stranded DNA sequence comprising a designated mutation is transcribed according to the given RT template, and any change of the DNA sequence in the genome located at the target site can be achieved.

Current studies showed that, the overall editing efficiency of PE is low and it requires improvements in various aspects. Improving the stability or expression of a PE protein by modifying an M-MLV protein or fusing with other related proteins is an effective way to improve the PE editing efficiency.

### Summary of the Invention

The present invention relates to improving the prime editing efficiency by i) point-mutating on a reverse transcriptase or deleting a redundant domain such as a RNase H domain from a reverse transcriptase; ii) fusing the reverse transcriptase with a nucleocapsid protein NC; or a combination of i) and ii).

The present invention at least includes the following embodiments:
Embodiment 1: A prime editing system for targeted modification of a plant genome, comprising:
   i) a prime editing fusion protein and/or an expression construct comprising a nucleotide sequence encoding the prime editing fusion protein, wherein the prime editing fusion protein comprises a CRISPR nickase and a reverse transcriptase; and/or
   ii) at least one pegRNA and/or an expression construct comprising a nucleotide sequence encoding the at least one pegRNA,

   wherein the at least one pegRNA comprises, in the direction from 5' to 3', a guide sequence, a scaffold sequence, a reverse transcription (RT) template sequence and a primer binding site (PBS) sequence,
   wherein the at least one pegRNA is capable of forming a complex with the fusion protein and targeting the fusion protein to a target sequence in the genome, so that a nick is formed in the target sequence.
Embodiment 2: The system of Embodiment 1, wherein the CRISPR nickase is a Cas9 nickase, e.g., comprising an amino acid sequence shown in SEQ ID NO: 2.
Embodiment 3: The system of Embodiment 1 or 2, wherein the reverse transcriptase is a M-MLV reverse transcriptase or a functional variant thereof.
Embodiment 4: The system of any one of Embodiments 1-3, wherein
   (a) the reverse transcriptase comprises a mutation selected from any one of F155Y, F155V, F156Y, D524N, D200C, wherein the amino acid position refers to SEQ ID NO: 3;
   (b) a connection sequence of the reverse transcriptase is deleted; and/or (c) a RNase H domain of the reverse transcriptase is mutated or deleted.
Embodiment 5: The system of Embodiment 4, wherein the reverse transcriptase comprises a sequence shown in any one of SEQ ID NOs: 9-15.
Embodiment 6: The system of any one of Embodiments 1-5, wherein the reverse transcriptase, such as the M-MLV reverse transcriptase, or the functional variant thereof is fused at N-terminus or C-terminus with a nucleocapsid protein (NC), a protease (PR), or an integrase (IN) directly or via a linker in the fusion protein.
Embodiment 7: The system of Embodiment 6, wherein the nucleocapsid protein (NC) contains an amino acid sequence shown in SEQ ID NO: 6, or the protease (PR) contains an amino acid sequence shown in SEQ ID NO: 7, or the integrase (IN) contains an amino acid sequence shown in SEQ ID NO: 8.
Embodiment 8: The system of Embodiment 6 or 7, wherein the reverse transcriptase is fused at the N-terminus with the nucleocapsid protein (NC) directly or via a linker, and preferably, the functional variant of the M-MLV reverse transcriptase of which the RNase H domain is deleted is fused at the N-terminus with the nucleocapsid protein (NC) directly or via a linker.
Embodiment 9: The system of any one of Embodiments 1-8, wherein the guide sequence of the pegRNA is configured to have sufficient sequence identity with the target sequence and thus is capable of binding to a complementary strand of the target sequence by base pairing so as to achieve sequence-specific targeting.
Embodiment 10: The system of any one of Embodiments 1-9, wherein the scaffold sequence of the pegRNA comprises a sequence shown in SEQ ID NO: 17.
Embodiment 11: The system of any one of Embodiments 1-10, wherein the primer binding sequence is configured to be complementary to at least a portion of the target sequence, and preferably the primer binding sequence is complementary to at least a portion of a 3' free single strand caused by the nick, in particular to a nucleotide sequence at the 3' end of the 3' free single strand.
Embodiment 12: The system of any one of Embodiments 1-11, wherein the primer binding sequence has a Tm (melting temperature) of about 18-52 °C, preferably about 24-36 °C, more preferably about 28-32 °C, and most preferably about 30 °C.
Embodiment 13. The system of any one of Embodiments 1-12, wherein the RT template sequence is configured to correspond to a sequence downstream of the nick and comprises a desired modification, and the modification comprises substitution, deletion and/or addition of one or more nucleotides.
Embodiment 14: The system of any one of Embodiments 1-13, wherein the prime editing fusion protein contains an amino acid sequence shown in SEQ ID NO: 19.
Embodiment 15: A method of producing a genetically modified plant, comprising introducing the prime editing system of any one of Embodiments 1-14 into at least one cell, thereby resulting in a modification in the target sequence in the genome of the at least one cell.

### Brief Description of the Drawings

Fig. 1 shows different modified PPE constructs.
Fig. 2 shows editing efficiency of the different constructs obtained by flow cytometry analysis and screened by a BFP-GFP reporting system in a rice protoplast.
Fig. 3 shows editing efficiency of the prime editing systems for different endogenous testing sites obtained by amplicon sequencing analysis after the co-transformation of the screened constructs PPE-NCv1, PPE-NCv2, and PPE-ΔRNase H having the improved editing efficiency with the pegRNAs into the rice protoplast cell .
Fig. 4 shows editing efficiency of the prime editing systems for different endogenous testing sites obtained by amplicon sequencing analysis after the co-transformation of the PPE-NCv1, PPE-ΔRNase H, and a construct ePPE constructed with a combination of both of a fused NC and a deleted RNase H with the pegRNAs into the rice protoplast cell, respectively.
Fig. 5 shows the expression of the PPE proteins having the improved editing efficiency in the rice protoplast.
Fig. 6 shows that the ePPE protein may effectively improve the editing efficiency of deleting or inserting a fragment greater than 15 bp.
Fig. 7 shows that the ePPE protein may effectively expand the scope of the prime editing system.
Fig. 8 shows the editing efficiency of ePPE in rice calli.
Fig. 9 shows creation of an herbicide resistant rice plant using ePPE.
Fig. 10 shows editing of ePE in a pig cell.
Fig. 11 shows ePPE does not significantly increase the efficiency of off-target editing in most of the rice endogenous testing sites and the editing efficiency of the prime editing systems for the different endogenous testing sites and predicted endogenous off-target sites obtained by the amplicon sequencing analysis.
Fig. 12 shows a complementary function of ePPE and base editing at the edited site.
Fig. 13 shows that a combination of ePPE with dual-pegRNA strategy and epegRNAs strategy further improves the efficiency of prime editing.

### Detailed Description of the Invention

### I. Definition

In the present invention, unless indicated otherwise, the scientific and technological terminologies used herein refer to meanings commonly understood by a person skilled in the art. Also, the terminologies and experimental procedures used herein relating to protein and nucleotide chemistry, molecular biology, cell and tissue cultivation, microbiology, immunology, all belong to terminologies and conventional methods generally used in the art. For example, the standard DNA recombination and molecular cloning technology used herein are well known to a person skilled in the art, and are described in details in the following references: Sambrook, J., Fritsch, E.F.and Maniatis, T., Molecular Cloning: A Laboratory Manual; Cold Spring Harbor Laboratory Press: Cold Spring Harbor, 1989. In the meantime, in order to better understand the present invention, definitions and explanations for the relevant terminologies are provided below.

As used herein, the term "and/or" encompasses all combinations of items connected by the term, and each combination should be regarded as individually listed herein. For example, "A and/or B" covers "A", "A and B", and "B". For example, "A, B, and/or C" covers "A", "B", "C", "A and B", "A and C", "B and C", and "A and B and C".

When the term "comprise" is used herein to describe the sequence of a protein or nucleic acid, the protein or nucleic acid may consist of the sequence, or may have additional amino acids or nucleotide at one or both ends of the protein or nucleic acid, but still have the activity described in this invention. In addition, those skilled in the art know that the methionine encoded by the start codon at the N-terminus of the polypeptide will be retained under certain practical conditions (for example, when expressed in a specific expression system), but does not substantially affect the function of the polypeptide. Therefore, when describing the amino acid sequence of specific polypeptide in the specification and claims of the present application, although it may not include the methionine encoded by the start codon at the N-terminus, the sequence containing the methionine is also encompassed, correspondingly, its coding nucleotide sequence may also contain a start codon; vice versa.

"Genome" as used herein encompasses not only chromosomal DNA present in the nucleus, but also organelle DNA present in the subcellular components (e.g., mitochondria, plastids) of the cell. As used in this article, "organism" includes any organisms suitable for genome editing, preferably a eukaryotic organism. Examples of the organisms include but not limited to mammals such as human, mouse, rat, monkey, dog, pig, sheep, cow, and cat; poultry such as chicken, duck, and geese; plants including monocotyledons and dicotyledons, such as rice, corn, wheat, sorghum, barley, soybean, peanut, and arabidopsis.

A "genetically modified organism" means an organism which comprises an exogenous polynucleotide or comprises a modified gene or expression regulatory sequence within its genome. For example, the exogenous polynucleotide can be stably integrated into the genome of the organism and inherited in successive generations. The exogenous polynucleotide may be integrated into the genome alone or as a part of a recombinant DNA construct. The modified gene or expression regulatory sequence is a gene or expression regulatory sequence comprising one or more nucleotide substitutions, deletions and additions in the organism genome.

"Polynucleotide", "nucleic acid sequence", "nucleotide sequence", or "nucleic acid fragment" are used interchangeably to refer to a polymer of RNA or DNA that is single- or double-stranded, optionally containing synthetic, non-natural or altered nucleotide bases. Nucleotides are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "D" for A, T or G, "I" for inosine, and "N" for any nucleotide.

"Polypeptide", "peptide", and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The terms "polypeptide", "peptide", "amino acid sequence", and "protein" are also inclusive of modifications including, but not limited to, glycosylation, lipid attachment, sulfation, gamma-carboxylation of glutamic acid residues, hydroxylation and ADP-ribosylation.

As used herein, an "expression construct" refers to a vector suitable for expression of a nucleotide sequence of interest in an organism, such as a recombinant vector. "Expression" refers to the production of a functional product. For example, the expression of a nucleotide sequence may refer to transcription of the nucleotide sequence (such as transcribe to produce an mRNA or a functional RNA) and/or translation of RNA into a protein precursor or a mature protein.

"Expression construct" of the invention may be a linear nucleic acid fragment, a circular plasmid, a viral vector, or, in some embodiments, an RNA that can be translated (such as an mRNA), such as an mRNA produced by transcription *in vitro.*

"Expression construct" of the invention may comprise regulatory sequences and nucleotide sequences of interest that are derived from different sources, or regulatory sequences and nucleotide sequences of interest derived from the same source, but arranged in a manner different than that normally found in nature.

"Promoter" refers to a nucleic acid fragment capable of controlling the transcription of another nucleic acid fragment. In some embodiments of the present invention, the promoter is a promoter capable of controlling the transcription of a gene in a cell, whether or not it is derived from the cell. The promoter may be a constitutive promoter or a tissue-specific promoter or a developmentally-regulated promoter or an inducible promoter.

Examples of promoters include but are not limited to polymerase (pol) I, pol II or pol III promoters. Examples of pol I promoters include chicken RNA pol I promoter. Examples of pol II promoters include but are not limited to cytomegalovirus immediate early (CMV) promoter, rous sarcoma virus long terminal repeat(RSV-LTR) promoter and simian virus 40(SV40) immediate early promoter. Examples of pol III promoters include U6 and H1 promoter. Inducible promoter such as metalothionein promoter can be used. Other examples of promoters include T7 bacteriophage promoter, T3 bacteriophage promoter, β-galactosidase promoter and Sp6 bacteriophage promoter etc. When used for plants, promoters that can be used include but are not limited to cauliflower mosaic virus 35S promoter, maize Ubi-1 promoter, wheat U6 promoter, rice U3 promoter, maize U3 promoter and rice actin promoter etc.

"Introduction" of a nucleic acid molecule (e.g., plasmid, linear nucleic acid fragment, RNA, etc.) or protein into an organism means that the nucleic acid or protein is used to transform a cell of the organism such that the nucleic acid or protein functions in the cell. As used in the present invention, "transformation" includes both stable and transient transformations. "Stable transformation" refers to the introduction of an exogenous nucleotide sequence into the genome, resulting in the stable inheritance of foreign genes. Once stably transformed, the exogenous nucleic acid sequence is stably integrated into the genome of the organism and any of its successive generations. "Transient transformation" refers to the introduction of a nucleic acid molecule or protein into a cell, performing its function without the stable inheritance of an exogenous gene. In transient transformation, the exogenous nucleic acid sequence is not integrated into the genome.

"Trait" refers to the physiological, morphological, biochemical, or physical characteristics of a cell or an organism.

### II. Improved prime editing system

In one aspect, the present invention relates to a prime editing system for targeted modification of an organism genome, comprising:
i) a prime editing fusion protein and/or an expression construct comprising a nucleotide sequence encoding the prime editing fusion protein, wherein the prime editing fusion protein comprises a CRISPR nickase and a reverse transcriptase; and/or
ii) at least one pegRNA and/or an expression construct comprising a nucleotide sequence encoding the at least one pegRNA,
wherein the at least one pegRNA comprises, in the direction from 5' to 3', a guide sequence, a scaffold sequence, a reverse transcription (RT) template sequence and a primer binding site (PBS) sequence.

In some embodiments, the CRISPR nickase and the reverse transcriptase in the prime editing fusion protein are linked via a linker.

In some embodiments, the at least one pegRNA is capable of forming a complex with the fusion protein and targeting the fusion protein to the target sequence in the genome, resulting in a nick in the target sequence.

In one aspect, the present invention relates to a prime editing fusion protein, wherein the prime editing fusion protein contains a CRISPR nickase and a reverse transcriptase.

In one aspect, the present invention relates to use of the prime editing fusion protein of the present invention in a targeted modification of a DNA sequence of an organism genome.

In some embodiments, the organism is a plant.

As used herein, a "prime editing system" refers to a combination of components required for reverse transcription-based genome editing of the genome in a cell. The individual components, e.g., the prime editing fusion protein, the gRNA, etc., of the system may be present independently of each other, or may be present in any combination as a composition.

As used herein, a "target sequence" refer to a sequence which is approximately 20 nucleotides in length in the genome and is represented by the 5' or 3' flanking PAM (protospacer adjacent motif) sequence. In general, PAM is required for recognizing the target sequence by the complex formed by the CRISPR nuclease or a variant thereof and the guide RNA. For example, for the Cas9 nuclease and a variant thereof, the target sequence is, at the 3' end, closely adjacent to the PAM , for example, 5'-NGG-3'. Based on the presence of PAM, those skilled in the art may readily determine the target sequences available in the genome for targeting. In addition, depending on the locations of the PAMs, the target sequences may be located on any strand of genome DNA molecule. For Cas9 or derivatives thereof such as the Cas9 nickase, the target sequences are preferably 20 nucleotides in length.

In some embodiments, the CRISPR nickase in the fusion protein is capable of forming a nick within the target sequence in the genome DNA. In some embodiments, the CRISPR nickase is a Cas9 nickase.

In some embodiments, the Cas9 nickase is derived from *Streptococcus pyogenes* (*S. pyogenes*) SpCas9, and comprises at least an amino acid substitution H840A relative to wild type SpCas9. The exemplary wild type SpCas9 comprises an amino acid sequence set forth in SEQ ID NO: 1. In some embodiments, the Cas9 nickase comprises an amino acid sequence set forth in SEQ ID NO: 2. In some embodiments, the Cas9 nickase in the fusion protein is capable of forming a nick between the -3 position nucleotide (the first nucleotide at the 5' end of each PAM sequence is considered as the +1 site nucleotide) and the -4 position nucleotide of the PAM of the target sequence.

In some embodiments, the Cas9 nickase is a Cas9 nickase variant capable of recognizing an altered PAM sequence. Various Cas9 nickase variants capable of recognizing an altered PAM sequence are known in the art. In some preferred embodiments, the Cas9 nickase is a Cas9 variant that recognizes the PAM sequence 5'-NG-3'. In some embodiments, the Cas9 nickase variant that recognizes the PAM sequence 5'-NG-3' comprises the following amino acid substitutions H840A, R1335V, L1111R, D1135V, G1218R, E1219F, A1322R and T1337R relative to the wild type Cas9, wherein the amino acid numbering refers to SEQ ID NO: 1.

The nick formed by the Cas9 nickase of the present invention is capable of causing the target sequence to form a free single strand at the 3' terminal (3' free single strand) and a free single strand at the 5' terminal (5' free single strand).

In some embodiments, the reverse transcriptase in the fusion protein of the present invention may be derived from different sources. In some embodiments, the reverse transcriptase is a virus-derived reverse transcriptase. For example, in some embodiments, the reverse transcriptase is an M-MLV reverse transcriptase or a functional variant thereof. An exemplary wild type M-MLV reverse transcriptase sequence is set forth in SEQ ID NO: 3.

In some embodiments, the reverse transcriptase, such as the M-MLV reverse transcriptase, or the functional variant thereof
(a) comprises a mutation at a position selected from any one of 155, 156, 200 and/or 524, such as comprises a mutation selected from any one of F155Y, F155V, F156Y, D524N, D200C or the combination thereof, wherein the amino acid position refers to SEQ ID NO: 3;
(b) a connection sequence is deleted; and/or
(c) a RNase H domain is mutated or deleted.

In some preferred embodiments, the reverse transcriptase, such as the M-MLV reverse transcriptase, or the functional variant thereof, contains a mutation selected from D524N, and the amino acid position refers to SEQ ID NO: 3.

In some preferred embodiments, the RNase H domain of the reverse transcriptase, such as the M-MLV reverse transcriptase, or the functional variant thereof, is deleted.

In some embodiments, the connection sequence contains an amino acid sequence shown in SEQ ID NO: 4.

In some embodiments, the RNase H domain contains an amino acid sequence shown in SEQ ID NO: 5.

In some embodiments, the reverse transcriptase, such as the M-MLV reverse transcriptase, or the functional variant thereof, contains a sequence shown in any one of SEQ ID NO: 9-15.

In some embodiments, the reverse transcriptase, such as the M-MLV reverse transcriptase, or the functional variant thereof is fused at N-terminus or C-terminus with a nucleocapsid protein (NC), a protease (PR), or an integrase (IN) directly or via a linker in the fusion protein. The nucleocapsid protein (NC), protease (PR), or integrase (IN), for example, is derived from M-MLV.

In some embodiments, the nucleocapsid protein (NC) contains an amino acid sequence shown in SEQ ID NO: 6.

In some embodiments, the protease (PR) contains an amino acid sequence shown in SEQ ID NO: 7.

In some embodiments, the integrase (IN) contains an amino acid sequence shown in SEQ ID NO: 8.

In some preferred embodiments, the reverse transcriptase, such as the M-MLV reverse transcriptase, or the functional variant thereof, is fused at the N-terminus with the nucleocapsid protein (NC) directly or via a linker.

In some preferred embodiments, the reverse transcriptase, such as the M-MLV reverse transcriptase, or the functional variant thereof, is fused at the C-terminus with the nucleocapsid protein (NC) directly or via a linker.

As used herein, a "linker" may be a non-functional amino acid sequence which is 1-50 (e.g. 1, 2, 3, 4, 5, 6, 7, 8, 9, 10, 11, 12, 13, 14, 15, 16, 17, 18, 19, 20 or 20-25 or 25-50) or more amino acids in length and is free of secondary or higher structures. For example, the linker may be a flexible linker, for example, GGGGS, GS, GAP, (GGGGS)x3, GGS, (GGS) x7, etc. For example, the linker may be a linker set forth in SEQ ID NO: 6.

In some embodiments, the CRISPR nickase in the fusion protein is located at the N-terminus of the reverse transcriptase. In some embodiments, the CRISPR nickase in the fusion protein is located at the C-terminus of the reverse transcriptase.

In some embodiments of the present invention, the fusion protein of the present invention may further comprise a nuclear localization sequence (NLS). In general, one or more NLSs in the fusion protein should be of sufficient strength to drive accumulation of the fusion protein in nuclei of the cells in an amount that can realize its editing function. In general, the strength of nuclear localization activity is determined by the number of NLS in the fusion protein, location of NLS in the fusion protein, the one or more specific NLSs as used in the fusion protein, or a combination of these factors.

In some embodiments, the fusion protein contains an amino acid sequence shown in SEQ ID NO: 19.

The guide sequence (also referred to as seed sequence or spacer sequence) in at least one pegRNA of the present invention is configured to have sufficient sequence identity (preferably 100% identity) to the target sequence so as to achieve sequence-specific targeting by binding to complementary strand of the target sequence through base pairing.

A variety of scaffold sequences of the gRNAs suitable for genome editing based on the CRISPR nuclease (e.g. Cas9) are known in the art and may be used in the pegRNA of the present invention. In some embodiments, the scaffold sequence of the gRNA is set forth in SEQ ID NO: 17.

In some embodiments, the primer binding sequence is configured to be complementary to at least a portion of the target sequence (preferably fully paired with at least a portion of the target sequence), and preferably, the primer binding sequence is complementary to (preferably fully paired with at least a portion of the 3' free single strand) at least a portion of the 3' free single strand caused by the nick in the DNA strand where the target sequence is located, in particular complementary to (preferably fully paired with) the nucleotide sequences at the 3' terminal of the 3' free single strand. When the 3' free single strand of the strand binds to the primer binding sequence by base pairing, the 3' free single strand can serve as a primer, and the reverse transcription (RT) template sequence closely adjacent to the primer binding sequence can serve as a template for reverse transcription by the reverse transcriptase in the fusion protein, and the DNA sequence corresponding to the reverse transcription (RT) template sequence is obtained through extension.

The primer binding sequence depends on the length of the free single strand formed in the target sequence by the CRISPR nickase, however, they should have a minimum length capable of ensuring specific binding. In some embodiments, the primer binding sequence may be 4-20 nucleotides in length, for example, 4, 5, 6, 7, 8, 9, 10, 11, 12, 13, 14, 15, 16, 17, 18, 19 and 20 nucleotides in length.

In some embodiments, the primer binding sequence is configured to have a Tm (melting temperature) of no more than about 52°C. In some embodiments, the Tm (melting temperature) of the primer binding sequences is about 18°C-52°C, preferably about 24°C-36°C, more preferably about 28°C-32°C, and most preferably about 30°C.

Methods for calculating the Tm of a nucleic acid sequence is well known in the art, for example, an Oligo Analysis Tool online analytical tool may be used for calculation. An exemplary calculation formula is Tm=N _{G:C} *< 4+N _{A:T} *< 2, wherein N _{G:C} is the number of G and C bases in the sequence, and N _{A:T} is the number of A and T bases in the sequence. The suitable Tm may be obtained by selecting a suitable PBS length. Alternatively, PBS sequence with the appropriate Tm may be obtained by selecting the appropriate target sequence.

In some embodiments, the RT template sequence may be any sequence. Through the above reverse transcription, sequence information thereof may be integrated into the DNA strand where the target sequence is located (namely, the strand containing the PAM of the target sequence), and then DNA double strands containing the sequence information of the RT template are formed through DNA repairing of the cell. In some embodiments, the RT template sequence comprises desired modification(s). For example, the desired modification comprises substitution, deletion and/or addition of one or more nucleotide. For example, the modification comprises one or more substitutions selected from: C to T substitution, C to G substitution, C to A substitution, G to T substitution, G to C substitution, G to A substitution, A to T substitution, A to G substitution, A to C substitution, T to C substitution, T to G substitution and T to A substitution; and/or comprises deletion of one or more nucleotides, for example, deletion of 1 to about 100 or more nucleotides, e.g. deletion of 1, 2, 3, 4, 5, about 10, about 20, about 30, about 40, about 50, about 75, about 100 nucleotides; and/or comprises insertion of one or more nucleotides, for example, insertion of 1 to about 100 or more nucleotides, for example, insertion of 1 to about 100 or more nucleotides, for example, insertion of 1, 2, 3, 4, 5, about 10, about 20, about 30, about 40, about 50, about 75, about 100 nucleotides.

In some embodiments, the RT template sequence is configured as corresponding to the sequence downstream the nick of the target sequence (e.g., being complementary to at least a portion of the sequence downstream the nick of the target sequence) and contain desired modification(s). For example, the desired modification comprises substitution, deletion and/or addition of one or more nucleotide. For example, the modification comprises one or more substitutions selected from: C to T substitution, C to G substitution, C to A substitution, G to T substitution, G to C substitution, G to A substitution, A to T substitution, A to G substitution, A to C substitution, T to C substitution, T to G substitution and T to A substitution; and/or comprises deletion of one or more nucleotides, for example, deletion of 1 to about 100 or more nucleotides, e.g. deletion of 1, 2, 3, 4, 5, about 10, about 20, about 30, about 40, about 50, about 75, about 100 nucleotides; and/or comprises insertion of one or more nucleotides, for example, insertion of 1 to about 100 or more nucleotides, for example, insertion of 1 to about 100 or more nucleotides, for example, insertion of 1, 2, 3, 4, 5, about 10, about 20, about 30, about 40, about 50, about 75, about 100 nucleotides.

In some embodiments, the RT template sequence may be about 1-300 or more nucleotides in length, for example, 1, 2, 3, 4, 5, about 10, about 20, about 30, about 40, about 50, about 75, about 100, about 125, about 150, about 175, about 200, about 225, about 250, about 275, about 300 nucleotides or more nucleotides in length. Preferably, the RT template sequence is 7, 8, 9, 10, 11, 12, 13, 14, 15, 16, 17, 18, 19, 20, 21, 22 and 23 nucleotides in length.

In some embodiments, the prime editing system further comprises a nicking gRNA (the nicking gRNA is used for generating an additional nick) and/or an expression construct comprising a nucleotide sequence encoding the nicking gRNA, wherein the nicking gRNA comprises a guide sequence and a scaffold sequence. In some preferred embodiments, the nicking gRNA does not contain the reverse transcription (RT) template sequence and the primer binding site (PBS) sequence.

The guide sequence (also referred to as seed sequence or spacer sequence) in the nicking gRNA of the present invention is configured to have sufficient sequence identity (preferably 100% identity) to a nicking target sequence in the genome, so that the fusion protein of the present invention is targeted to the nicking target sequence, resulting in a nick in the nicking target sequence, wherein the nicking target sequence and the target sequence (pegRNA target sequence) targeted by the pegRNA are located on the opposite strand of the genome DNA. In some embodiments, the nick formed by the nicking RNA and the nick formed by the pegRNA are about 1 to about 300 or more nucleotides apart, e.g., 1, 2, 3, 4, 5, about 10, about 20, about 30, about 40, about 50, about 75, about 100, about 125, about 150, about 175, about 200, about 225, about 250, about 275, about 300 nucleotides or more nucleotides apart. In some embodiments, the nick created by the nicking RNA is located upstream or downstream of the nick formed by the pegRNA (upstream or downstream refers to the DNA strand on which the pegRNA target sequence is located). In some embodiments, the guide sequence in the nicking gRNA has sufficient sequence identity (preferably 100% identity) to the pegRNA target sequence on the opposite strand after editing event occur (modified pegRNA target sequence), so that the nicking gRNA targets only the nicking target sequence that is generated after pegRNA-induced sequence targeting and modification. In some embodiments, the PAM of the nicking target sequence is located within the complementary sequence of the pegRNA target sequence.

In some embodiments, the sequence of the pegRNA and/or the nicking gRNA may be precisely processed by using a self-processing system. In some specific embodiments, the 5' end of the pegRNA and/or the nicking gRNA are linked to the 3' end of a first ribozyme, wherein the first ribozyme is designed to cleave the fusion at the 5' end of the pegRNA and/or the nicking gRNA; and/or the 3' end of the pegRNA and/or the nicking gRNA is linked to the 5' end of a second ribozyme, wherein the second ribozyme is designed to cleave the fusion at the 3' end of the pegRNA and/or the nicking gRNA. The design of the first or second ribozyme is within the capabilities of those skilled in the art. For example, see Gao et al. JIPB, apr, 2014; Vol 56, Issue 4, 343-349. As for a method for precise processing of gRNA, see, for example, WO 2018/149418.

In some embodiments, the prime editing system comprises at least one pair of pegRNAs and/or expression construct comprising the nucleotide sequence encoding the at least one pair of pegRNAs. In some embodiments, the two pegRNAs of the pair of pegRNAs are configured to target different target sequences on the same strand of the genome DNA. In some embodiments, the two pegRNAs of the pair of pegRNAs are configured to target the target sequences on different strands of the genome DNA. In some embodiments, the PAM for the target sequence of one pegRNA of the pair of pegRNAs is located on a sense strand, and the PAM for the other pegRNA is located on an antisense strand. In some embodiments, the induced nicks of the two pegRNAs are located on both sides of a site to be modified respectively. In some embodiments, the pegRNA-induced nick for the sense strand is located upstream (5' direction) of the site to be modified, and the pegRNA-induced nick for the antisense strand is located downstream (3' direction) of the site to be modified. The "upstream" or "downstream" refers to the sense strand. In some embodiments, the nicks induced by the two pegRNAs are about 1 to about 300 or more nucleotides apart, for example, 1-15 nucleotides apart.

In some embodiments, the two pegRNAs in the pair of pegRNAs are configured to introduce the same desired modification. For example, one pegRNA is configured to introduce a A to G substitution in the sense strand, and the other pegRNA is configured to introduce a T to C substitution at the corresponding position of the antisense strand. For another example, one pegRNA is configured to introduce two nucleotide deletions in the sense strand, and the other pegRNA is configured to similarly introduce two nucleotide deletions at the corresponding position of the antisense strand. Other types of modification may be made in a similar manner. Designing appropriate RT template sequences can allow the pegRNAs targeting two different strands to achieve the same desired modification.

In some preferred embodiments, the pegRNA in the present invention is epegRNA. The construction of epegRNA may refer to James W. Nelson et al., Engineered pegRNAs improve prime editing efficiency. Nature Biotechnology volume 40, 402-410 (2022), which is incorporated by reference herein. In some embodiments, the epegRNA is an epegRNA with a 3'-tevopreQ1-8nt linker modification.

In order to obtain efficient expression in different organisms, in some embodiments of the present invention, the nucleotide sequence encoding the fusion protein is subjected to codon optimization for organism species whose genome is to be modified.

Codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at www.kazusa.orjp/codon/ and these tables can be adapted in a number of ways. See Nakamura, Y, et al. "Codon usage tabulated from the international DNA sequence databases: status for the year 2000" Nucl. Acids Res. 28:292 (2000).

### III. Method for modifying target sequence in cell genome

In another aspect, the present invention provides a method of producing a genetically modified cell, comprising introducing the prime editing system of the present invention into at least one cell, resulting in modification in a target sequence in the genome of the at least one cell. The modification comprises substitution, deletion and/or addition of one or more nucleotide. For example, the modification comprises one or more substitutions selected from: C to T substitution, C to G substitution, C to A substitution, G to T substitution, G to C substitution, G to A substitution, A to T substitution, A to G substitution, A to C substitution, T to C substitution, T to G substitution and T to A substitution; and/or comprises deletion of one or more nucleotides, for example, deletion of 1 to about 100 or more nucleotides, e.g. deletion of 1, 2, 3, 4, 5, about 10, about 20, about 30, about 40, about 50, about 75, about 100 nucleotides; and/or comprises insertion of one or more nucleotides, for example, insertion of 1 to about 100 or more nucleotides, for example, insertion of 1 to about 100 or more nucleotides, for example, insertion of 1, 2, 3, 4, 5, about 10, about 20, about 30, about 40, about 50, about 75, about 100 nucleotides.

In another aspect, the present invention also provides a method of producing a genetically modified cell, comprising introducing the prime editing system of the present invention into the cell.

In another aspect, the present invention also provides a genetically modified organism, containing a genetically modified cell produced by the method of the present invention, and progenies of the cell.

In the present invention, the target sequence to be modified may be located at any location in the genome, for example, in a functional gene such as a protein-encoding gene, or may be, for example, located in a gene expression regulatory region such as a promoter region or an enhancer region, thereby the gene functional modification or gene expression modification can be achieved. Modifications in the target sequence of the cell can be detected by T7EI, PCR/RE or sequencing methods.

In the methods of the present invention, the prime editing system can be introduced into cells by a variety of methods well known to those skilled in the art.

Methods that can be used to introduce a prime editing system of the present invention into a cell include, but are not limited to, calcium phosphate transfection, protoplast fusion, electroporation, lipofection, microinjection, viral infection (e.g., baculovirus, vaccinia virus, adenovirus, adeno-associated virus, lentivirus and other viruses), gene gun method, PEG-mediated protoplast transformation, Agrobacterium-mediated transformation.

The cell that may be genetically edited by the method of the present invention may be derived from, for example, mammals such as human, mouse, rat, monkey, dog, pig, sheep, cow, and cat; poultry such as chicken, duck, and geese; and plants including monocotyledons and dicotyledons, such as rice, corn, wheat, sorghum, barley, soybean, peanut, and arabidopsis.

In some embodiments, the method of the invention is performed in vitro. For example, the cell is an isolated cell, or a cell in an isolated tissue or organ.

In some other embodiments, the method of the present invention can also be performed in vivo. For example, the cell is a cell within an organism, and the system of the invention can be introduced in vivo by, for example, a virus or Agrobacterium-mediated method.

### IV Method for producing genetically modified plant

In another aspect, the present invention provides a method of producing a genetically modified plant, comprising introducing the prime editing system of the present invention into at least one plant, resulting in modification in genome of the at least one plant. The modification comprises substitution, deletion and/or addition of one or more nucleotide. For example, the modification comprises one or more substitutions selected from: C to T substitution, C to G substitution, C to A substitution, G to T substitution, G to C substitution, G to A substitution, A to T substitution, A to G substitution, A to C substitution, T to C substitution, T to G substitution and T to A substitution; and/or comprises deletion of one or more nucleotides, for example, deletion of 1 to about 100 or more nucleotides, e.g. deletion of 1, 2, 3, 4, 5, about 10, about 20, about 30, about 40, about 50, about 75, about 100 nucleotides; and/or comprises insertion of one or more nucleotides, for example, insertion of 1 to about 100 or more nucleotides, for example, insertion of 1 to about 100 or more nucleotides, for example, insertion of 1, 2, 3, 4, 5, about 10, about 20, about 30, about 40, about 50, about 75, about 100 nucleotides.

In some embodiments, the method further comprises the step of screening the plant with the desired modification from the at least one plant.

In the method of the present invention, the prime editing system may be introduced into the plants in a variety of methods well known to those skilled in the art. The methods used for introducing the genome editing system of the present invention into a plant include, but are not limited to a gene gun method, PEG-mediated protoplast transformation, Agrobacterium-mediated transformation, plant virus-mediated transformation, pollen tube pathway and ovary injection method. Preferably, the prime editing system is introduced into the plant by transient transformation.

In the method of the present invention, modification of the genome may be achieved by only introducing or producing the prime editing fusion protein and the gRNAs in the plant cells, and the modification can be stably inherited without stably transforming exogenous polynucleotides encoding the components of the editing system into the plant. This avoids the potential off-target effects due to the stably existing (continuously produced) genome editing system and also avoids the integration of the exogenous nucleotide sequences in the plant genome, thereby providing greater biosafety.

In some preferred embodiments, the introduction is carried out in the absence of selection pressure to avoid integration of the exogenous nucleotide sequence into the plant genome.

In some embodiments, the introduction comprises transforming the genome editing system of the present invention into an isolated plant cell or tissue and then regenerating the transformed plant cell or tissue into an intact plant. Preferably, the regeneration is carried out in the absence of selection pressure, i.e., no selection agent for the selectable gene on the expression vector is used during tissue culture. Avoiding the use of a selection agent can increase the regeneration efficiency of the plant, obtaining a modified plant free of exogenous nucleotide sequences.

In other embodiments, the prime editing system of the present invention can be transformed into specific parts of an intact plant, such as leaves, shoot tips, pollen tubes, young ears or hypocotyls.

This is particularly suitable for the transformation of plants that are difficult to regenerate in tissue culture.

In some embodiments of the invention, the *in vitro* expressed protein and/or the in vitro transcribed RNA molecule (e.g., the expression construct is *in vitro* transcribed RNA molecule) are directly transformed into the plant. The protein and/or RNA molecule is capable of performing genome editing in plant cells and is subsequently degraded by the cell, avoiding integration of the exogenous nucleotide sequence in the plant genome.

Thus, in some embodiments, using the method of the present invention for plant genetic modification and breeding can obtain plants with the genome free of exogenous polynucleotide integration, namely transgene-free modified plants.

In some embodiments, the method further comprises culturing the plant cell, tissue or whole plant into which the prime editing system has been introduced at an elevated temperature, for example, the elevated temperature is 37°C.

In some embodiments of the present invention, the modified genome region is associated with plant traits, such as the agronomic traits, so that modified results in a plant having altered (preferably improved) traits, such as agronomic traits, relative to a wild type plant.

In some embodiments, the method further comprises the step of screening a plant having the desired modification and/or desired traits, such as agronomic traits.

In some embodiments of the present invention, the method further comprises obtaining progenies of the genetically modified plant. Preferably, the genetically modified plant or the progenies thereof have the desired modification and/or the desired traits, such as agronomic traits.

In another aspect, the invention also provides a genetically modified plant or progenies or a part thereof, wherein the plant is obtained by the method according to the present invention as described above. In some embodiments, the genetically modified plant or the progenies or a part thereof is transgene-free. Preferably, the genetically modified plant or progenies thereof have the desired genetic modification and/or the desired traits, such as agronomic traits.

In another aspect, the present invention provides a method of plant breeding comprising crossing a first genetically modified plant obtained by the above method of the present invention with a second plant not containing the modification, thereby the genetic modification is introduced into the second plant. Preferably, the first genetically modified plant has the desired traits, such as the agronomic traits.

"Agronomic trait" is a measurable parameter including but not limited to, leaf greenness, yield, growth rate, biomass, fresh weight at maturation, dry weight at maturation, fruit yield, seed yield, total plant nitrogen content, fruit nitrogen content, seed nitrogen content, nitrogen content in a vegetative tissue, total plant free amino acid content, fruit free amino acid content, seed free amino acid content, free amino acid content in a vegetative tissue, total plant protein content, fruit protein content, seed protein content, protein content in a vegetative tissue, drought tolerance, nitrogen uptake, root lodging, harvest index, stalk lodging, plant height, ear height, ear length, disease resistance, cold resistance, salt tolerance, and tiller number and so on.

### V Kit

The invention also includes a kit for use in the methods of the invention, and the kit at least comprises the prime editing system of the invention or the expression construct of the prime editing fusion protein. The kit also contains an expression construct for preparing pegRNA and a related reagent. The kit may also include a reagent for introducing the prime editing system into an organism or an organism cell. The kit also includes a label indicating the intended use and/or method of use of the contents in the kit. The term label includes any written or recorded material provided on or with the kit or otherwise provided with the kit.

### Example

Material and method

### 1. Vector construction

Gene fragments encoding PPE-F155Y, PPE-F155V, PPE-F156Y, PPE-D524N, PPE-N200C, PPE-ΔRNase H, and PPE-ΔRNase H-ΔConnection were obtained by point mutation or deletion of corresponding fragment at the corresponding site of the M-MLV reverse transcriptase in the gene fragment encoding PPE (SEQ ID NO: 18) by a polymerase chain reaction (PCR) respectively, and then, the resultant fragments were linked by a Gibson method, to obtain the corresponding final plasmids. The M-MLV reverse transcriptase sequences containing F155Y, F155V, F156Y, D524N, and N200C were shown in SEQ ID NO: 9-13 respectively. The sequence of M-MLV reverse transcriptase ΔRNase H was shown in SEQ ID NO: 14. The sequence of M-MLV reverse transcriptase ΔRNase H-ΔConnection was shown in SEQ ID NO: 15.

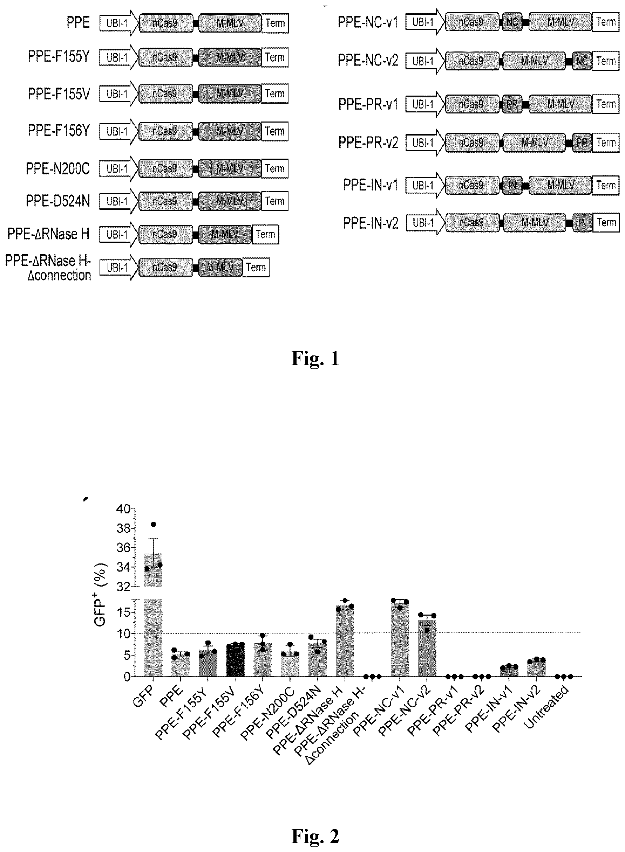

In addition, the gene fragment encoding nucleocapsid protein NC (SEQ ID NO: 6), protease PR (SEQ ID NO: 7), or integrase IN (SEQ ID NO: 8) were codon-optimized for plant and fused into 5'-terminal or 3'-terminal of the gene fragment encoding M-MLV of PPE respectively, and then, the resultant fragments were constructed by a Gibson method, to obtain the corresponding final plasmids. Similarly, the gene fragment encoding nucleocapsid protein NC was codon-optimized for plant and fused into 5'-terminal of the gene fragment encoding M-MLV reverse transcriptase ΔRNase H of PPE-ARNase H, to obtain PPE-ΔRNase H-NC (enhanced PPE, ePPE, and the amino acid sequence as shown in SEQ ID NO: 19). Subsequently, it was constructed by the Gibson method, to obtain the corresponding final vector. The specific vector was shown in Fig. 1.

The pegRNA fragments (including the RT and PBS sequences) were constructed into the vectors driven by *OsU3* promoter by using a Gibson method to obtain OsU3-pegRNA constructs suitable for use in rice.

### 2. Protoplast isolation and transformation

The protoplasts used in the present invention were derived from the rice variety Zhonghua 11.

### 2.1 Cultivation of rice seedlings

The rice variety was Zhonghua 11. Seeds were first rinsed with 75% ethanol for 1 min, then treated with 4% sodium hypochlorite for 30 min, and washed with sterile water for more than 5 times. The treated seeds were cultured on a M6 culture medium for 3-4 weeks at 26 °C, and protected from light.

### 2.2 Isolation of rice protoplasts

(1) Rice stalks were cut off and the middle parts thereof were cut into 0.5-1 mm filaments with a blade. The filaments were treated with a 0.6 M mannitol solution for 10 min in the dark, filtered with a filter, put into 50 mL an enzymolysis solution (filtered with a 0.45 µm membrane), vacuumized (pressure intensity of about 15 Kpa) for 30 min, taken out and placed on a shaker (10 rpm) for enzymolysis at room temperature for 5 h.
(2) The enzymolysis product was diluted by adding 30-50 mL W5 and filtered with a 75 µm nylon filter membrane into a round-bottomed centrifuge tube (50 mL).
(3) Centrifugation was conducted at 23 °C for 3 min at 250 g (rcf) prior to discarding the supernatant.
(4) The cells were slightly suspended with 20 mL W5, and step (3) was repeated.
(5) An appropriate amount of MMG was added to suspend the cells for transformation.

### 2.3 Transformation of protoplasts

(1) 10 µg vector to be transformed was added to each 2 mL centrifuge tube and mixed well. 200 µL protoplasts were taken with a tipped pipette and mixed well by flicking. 220 µL PEG4000 solution was added and mixed well by flicking, followed by inducing transformation for 20-30 min at room temperature in the absence of light.
(2) 880 µL W5 was added and mixed well by slight inverting, and centrifugation was conducted at 250 g (rcf) for 3 min prior to discarding the supernatant.
(3) 1 mL WI solution was added and mixed well by gently inverting before being gently transferred to a flow tube and cultured in the dark for about 40h at room temperature.

### 3. Flow cytometry analysis

The FACSAria III (BD Biosciences) instrument for the flow cytometry analysis has the following specific operation steps:
(1) It was checked whether there was sufficient liquid in the sheath tank and the ethanol tank and the waste tank was emptied. A stabilized voltage supply was turned on, the instrument was turned on, the fluorescent switch was tuned on, and BD FACSDiva Software was started, to enter a startup procedure.
(2) The air and fluid lines are disconnected, and then connected to the sheath tank, a closed-loop nozzle was verified to be in a corresponding position of the flow cell, and startup process was performed.
(3) The nozzle with a suitable size was replaced, the software mode was set to match the actual nozzle, the laser was turned on, and the stream was started.
(4) "New Protocol" was clicked to create a suitable experimental protocol.
(5) "Density plot" was selected to draw a FSC/SSC scatter diagram and a GFP/PE-Texas Red scatter diagram.
(6) The FSC/SSC voltage was adjusted to make the dots appear in the center of the scatter diagram. The FL1 voltage was adjusted to make the wild-type control protoplast population appear in the center of the scatter diagram, and the protoplast population of the experimental group (such as the protoplast transformed with GFP fluorescence protein) will appear in the location where the GFP fluorescence channel signal is higher. If necessary, compensated regulation was made, so that the two populations are more distinct.
(7) The threshold of the gate was determined based on the control group, ensuring that the protoplast populations in the experimental group were located within the threshold and the protoplast populations in the control group were located outside the threshold.
(8) The cell population to be sorted was clicked, the "left sort" was selected, and the analysis condition and the analysis mode were set according to the experimental needs and the percentage of target cells.
(9) The samples were loaded in proper order to read the data, and the relevant data was recorded.
(10) The stream was turned off, the closed-loop nozzle was replaced, the flow cell was cleaned with FACS clean liquid and sterile water, the software and the instrument were turned off, the stabilized voltage supply was turned off, and the instrument pressure was vented.

### 4. Protoplast DNA extraction and amplicon sequencing analysis

### 4.1 Protoplast DNA extraction

The protoplasts were collected in a 2 mL centrifuge tube, and protoplast DNA (~30 µL) with a concentration (30-60 ng/µL) measured by a NanoDrop ultra-micro spectrophotometer was extracted by a CTAB method and stored at -20 °C.

### 4.2 Amplicon Sequencing Analysis

(1) PCR amplification was conducted on a protoplast DNA template using genomic primers. A 20 µL amplification system comprised 4 µL 5xFastpfu buffer, 1.6 µL dNTPs (2.5 mM), 0.4 µL Forward primer (10 µM), 0.4 µL Reverse primer (10 µM), 0.4 µL FastPfu polymerase (2.5U/µL), and 2 µL DNA template (~60 ng). The amplification condition was as followings: pre-denaturation at 95 °C for 5 min; denaturation at 95 °C for 30 s, annealing at 50-64 °C for 30 s, extension at 72 °C for 30 s, for 35 cycles; fully extending at 72 °C for 5 min, and storing at 12 °C.
(2) The above amplification product was diluted by 10 times, 1 µL amplification product was taken as a second PCR amplification template, and the amplification primers were sequencing primers containing Barcode. A 50 µL amplification system comprised 10 µL 5× Fastpfu buffer, 4 µL dNTPs (2.5 mM), 1 µL Forward primer (10 µM), 1 µL Reverse primer (10 µM), 1 µL FastPfu polymerase (2.5U/µL). and 1 µL DNA template. The amplification condition was the same as that mentioned above, and the number of amplification cycles was 35 cycles.
(3) The PCR product was separated by 2% agarose gel electrophoresis, and gel extraction was conducted on a target fragment using the AxyPrep DNA Gel Extraction kit, and the resulting product was quantitatively analyzed using a NanoDrop ultra-micro spectrophotometer. The 100 ng recovered product was taken, mixed and sent to the Sangon Bioengineering Co., Ltd. for amplicon sequencing library construction and amplicon sequencing analysis.
(4) After sequencing was completed, the original data were split according to the sequencing primers, and WT was used as a control to compare and analyze the editing type and editing efficiency of the products at different genetic target loci in three repeated trials.

### 5. Protoplast protein extraction and Western-Blot

### 5.1. Protoplast protein extraction

(1) A protoplast sample after 48 h of culture was prepared and mixed uniformly by inverting, and centrifuged at 12000 rpm for 8 min. The supernatant was decanted, and 50 µL of the freshly prepared protein extraction solution was added, and was mixed uniformly by vortexing.
(2) The 1.5 mL centrifuge tube obtained from the previous step was placed on ice for 30 min, then was mixed uniformly by vortexing.
(3) The 1.5 mL centrifuge tube was centrifuged on a low-temperature centrifuge at 12000 rpm for 15 min at 4°C.
(4) The supernatant was pipetted with a pipette, and transferred to a new 1.5 mL centrifuge tube.
(5) 2 µL was pipetted from the above centrifuge tube and transferred to a new 1.5 mL centrifuge tube, and diluted by adding 18 µL of double distilled water.
(6) 1 mL of Bradford 1×Dye Reagent was added to the above system and placed for 5 min after mixing uniformly by vortexing, and the protein concentration was preliminarily measured with Eppendorf Biophotometer plus nucleic acid protein photometer.
(7) The concentrations of different samples were adjusted by the protein extraction buffer, 10 µL of the loading buffer was added and mixed uniformly by vortexing, and denaturized in a 100°C metal bath for 5 min.
(8) The resultant was centrifuged at 12000 rpm for 1 min, the supernatant was pipetted with a pipette, and subjected to SDS-PAGE gel electrophoresis.

### 5.2. Western-Blot

(1) 8% lower layer separating gel and 5% upper layer stacking gel were prepared. The samples were loaded, and electrophoresis was performed at 120 V for 1.5 h.
(2) NC membrane and filter paper were cut in advance, and immersed fully with a membrane transfer buffer.
(3) The transfer-to-membrane operation was performed with the semi-dry method. The immersed filter paper, the immersed NC membrane, the SDS-PAGE gel obtained after electrophoresis, and the immersed filter paper were placed from bottom to top. A 50 mL round bottom centrifuge tube was used to roll back and forth to expel all air bubbles.
(4) A constant current of 200 mA was applied for 1 h for protein transfer.
(5) The NC membrane was placed in a TBST buffer to wash off the membrane transfer buffer with shaking on a shaker at room temperature for 5 min.
(6) The buffer was decanted, an appropriate amount of 5% non-fat milk (formulated with TBST) was added, and the membrane was slowly shaken and incubated at room temperature on a shaker for 2 h for blocking.
(7) The blocking solution was decanted, a primary antibody diluted with an appropriate ratio was added to fresh 5% non-fat milk, and the membrane was incubated overnight on the shaker at 4°C at a low speed.
(8) The primary antibody was decanted, the NC membrane was washed with TBST buffer 3 times for 5 min each.
(9) Milk containing a diluted corresponding secondary antibody was added, and the membrane was incubated at room temperature for hours.
(10) The secondary antibody was decanted, the NC membrane was washed with TBST buffer 3 times for 5 min each, and the NC membrane was sealed in a specific plastic bag.
(11) The same amounts of developing solution A and developing solution B (Tiangen) were mixed uniformly, added to the above plastic bag, and the membrane was incubated for 3 min.
(12) The developing solution was decanted from the plastic bag, which was then placed in a dark folder, and developed in a dark room, and the developing time was adjusted according to the protein abundance.

### Example 1: Screening of different constructs with reporter system

In order to quickly and visually screen a construct with improved efficiency from the above multiple modifications, preliminary screening was conducted with a BFP-GFP reporter system in a rice protoplast. By flow cytometry analysis, the results were shown in Fig. 2, indicating that the editing efficiency of PPE-ΔRNase H, PPE-NCv1, and PPE-NCv2 were improved relative to original PPE. Therefore, these three constructs were selected for a follow-up testing on endogenous target.

### Example 2: Improvement of editing efficiency of prime editing system with different PPE protein modifications

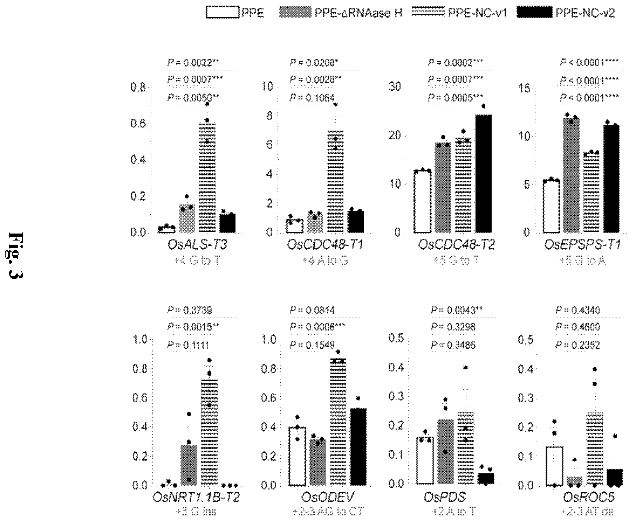

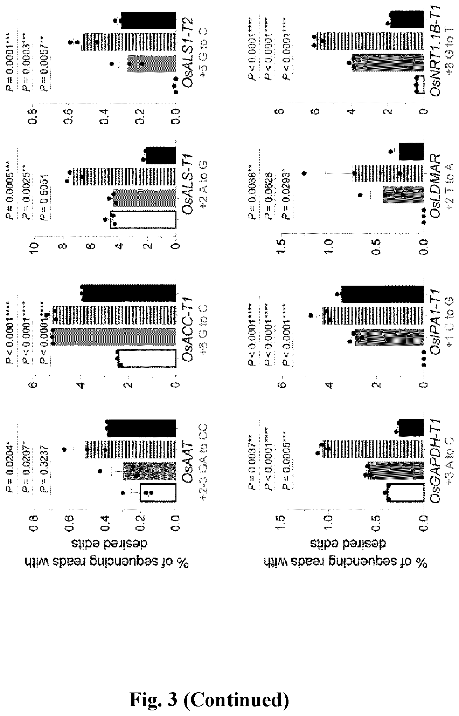

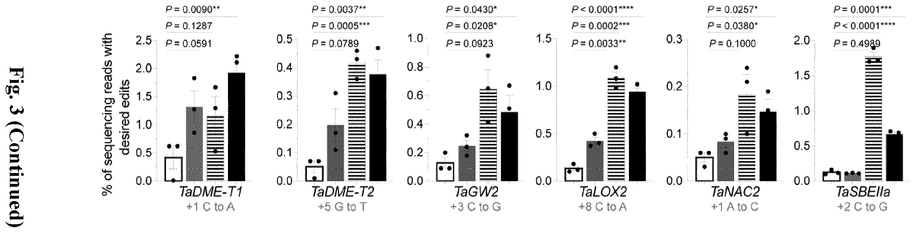

In order to test the effects of the above selected constructs on the prime editing system, 16 endogenous targets of rice were tested. These constructs were co-transformed into a rice protoplast cell with pegRNA, and the editing efficiency of the prime editing system was analyzed by amplicon sequencing analysis. The results were shown in Fig. 3, indicating that in most testing sites, PPE-NCv1, PPE-NCv2, and PPE-ΔRNase H had different degrees of improvement on the efficiency as compared with PPE. PPE-NCv1 achieved the highest improvement degree, while PPE-NCv2 and PPE-ΔRNase H taken second place.

### Example 3: Further improvement of editing efficiency of prime editing system by combination of different PPE protein modifications

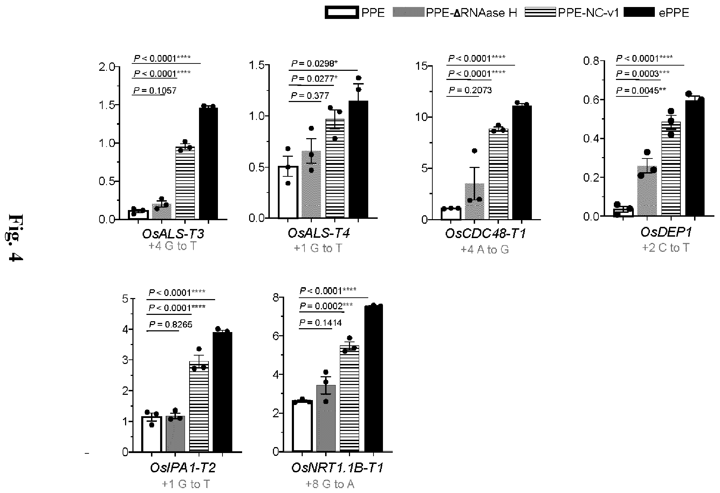

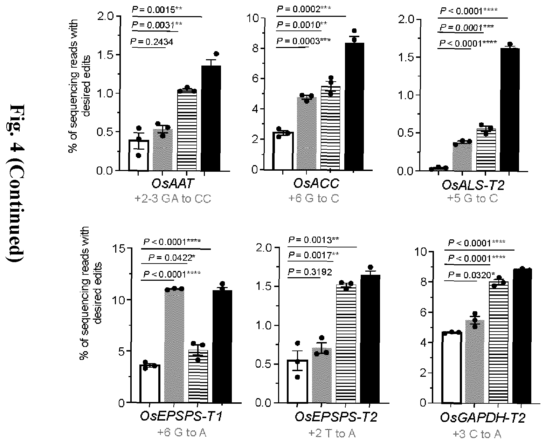

In order to test whether the efficiency of the prime editing system might be further improved by the combination of the fusion with NC and the deletion of RNase H (ePPE), 12 sites were selected for testing. These constructs were co-transformed into a rice protoplast cell with pegRNA, and the editing efficiency of the prime editing system was analyzed by amplicon sequencing analysis. The results were shown in Fig. 4, indicating that in most sites, ePPE(PPE-NCv1+ΔRNase H) had the highest editing efficiency, and the efficiency from high to low in order was ePPE>PPE-NCv1>PPE-ΔRNase H>PPE.

### Example 4: Testing of protein expression of different modified PPE protein

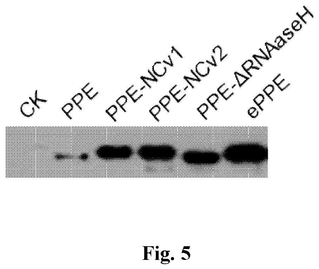

In order to test whether the above modified PPE protein had the increased protein expression compared to the original PPE protein and resulting in an improved prime editing efficiency, the corresponding protein was transformed into a rice protoplast, and a protoplast protein was extracted for Western Blot. The results were shown in Fig. 5, indicating that the expression of PPE-NCv1, PPE-NCv2, PPE-ΔRNase H, and ePPE proteins were significantly higher than that of the original PPE protein, and the expression of the ePPE protein was the highest, corresponding to its prime editing efficiency.

### Example 5: ePPE protein may effectively improve the editing efficiency of deleting or inserting a fragment larger than 15 bp

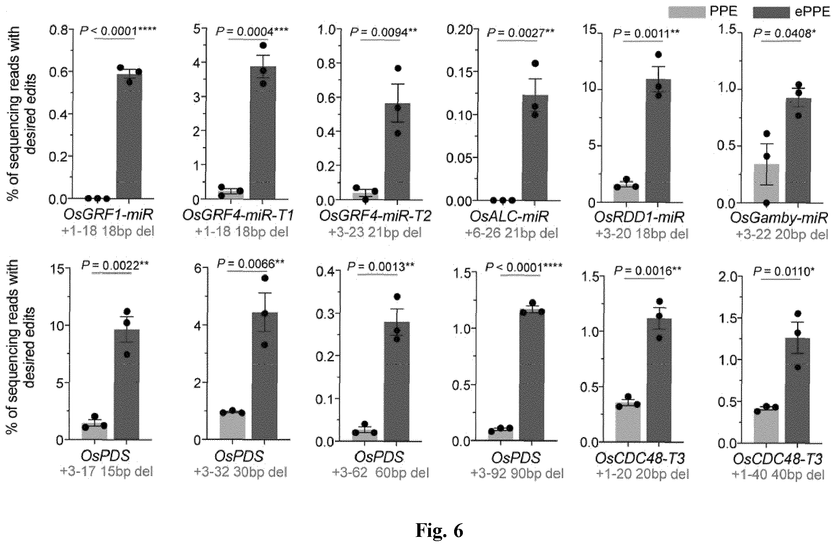

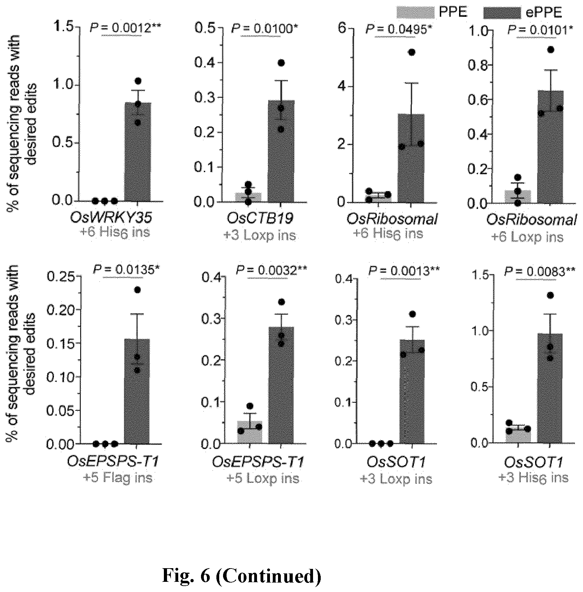

In view that the prime editing system is effective on base substitution or deletions or insertion of a few base, we further tested the efficiency of ePPE on insertion or deletion of larger fragment compared to the original PPE protein. 18 sites were selected for testing, and these constructs were co-transformed into a rice protoplast cell with pegRNA, and the editing efficiency of the prime editing system was analyzed by amplicon sequencing analysis. The results were shown in Fig. 6, indicating that in the tested sites, the editing efficiency of ePPE was the highest, and it might effectively achieve insertion of a fragment having 18-34 bp of and deletion of a fragment having 15-90 bp.

### Example 6: ePPE protein may effectively expand the scope of the prime editing system

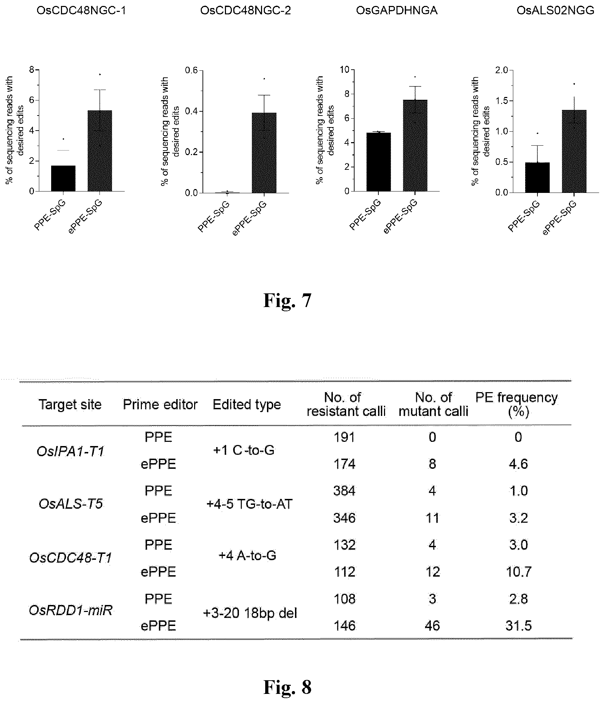

In order to test the targeting effect of ePPE fused with a SpG nuclease on other PAMs compared to the original PPE protein, NGC, NGA, NGG PAM of 4 sites were selected for testing, these constructs were co-transformed into a rice protoplast cell with pegRNA, and the editing efficiency of the prime editing system was analyzed by amplicon sequencing analysis. The results were shown in Fig. 7, and the editing efficiency of ePPE might effectively expand the prime editing effect on the tested sites.

### Example 7: Editing efficiency of ePPE in rice calli

In order to further test the mutation efficiency of ePPE in stable transformed rice calli, four targets in rice were selected for testing. After identification of resistant calli, the results were shown in Fig. 8, the editing efficiency of ePPE in the calli was consistent with its efficiency tested in the protoplast, both of which were higher than the editing efficiency of the control PPE, and the editing efficiency might be up to 31.5%.

### Example 8: Generation of herbicide resistant rice plant by ePPE

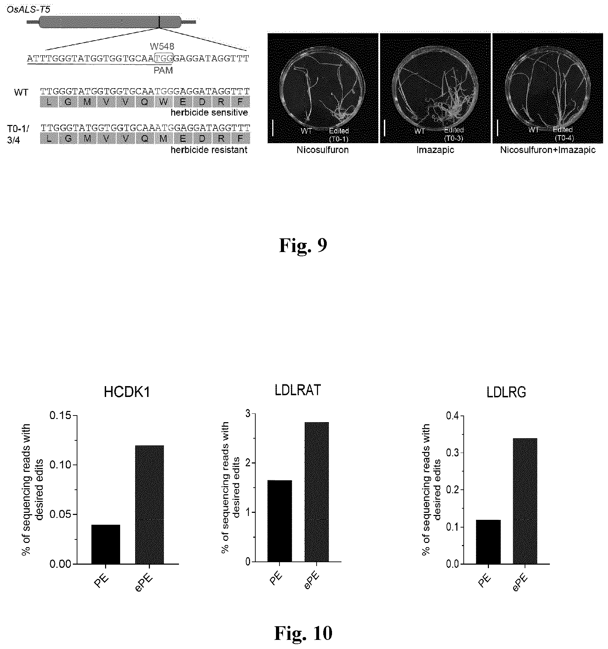

By targeting W548 site of a rice *OsALS* gene to produce a mutation of W548M, it was found that the mutation efficiency mediated by ePPE was 11.3%, while the efficiency mediated by PPE was only 0.6%. A heterozygous mutant was tested for herbicide resistance, and it was found that it had well resistance to nicosulfuron and imazapic, as well as a combination thereof. The results were shown in Fig. 9.

### Example 9: Editing in pig cell with ePE

In order to test the editing efficiency of ePPE in other species, 3 targets of a pig cell were selected for testing (named ePE). The results were shown in Fig. 10. ePE indeed showed different degrees of efficiency improvement compared to treatment with the original PE.

### Example 10: Off-target phenomenon of ePPE protein in prime editing

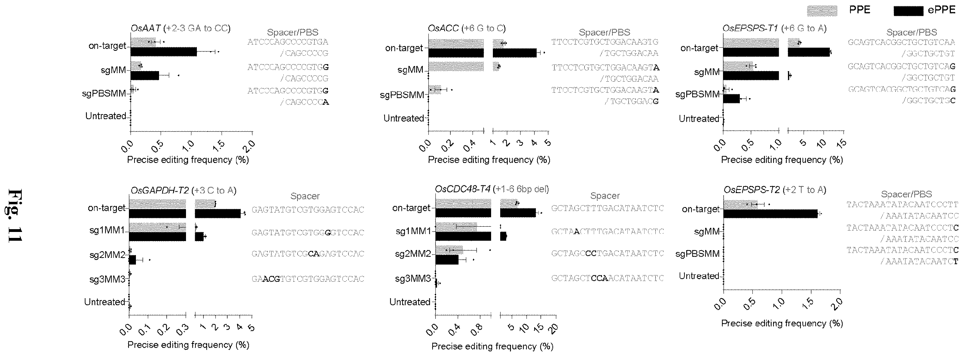

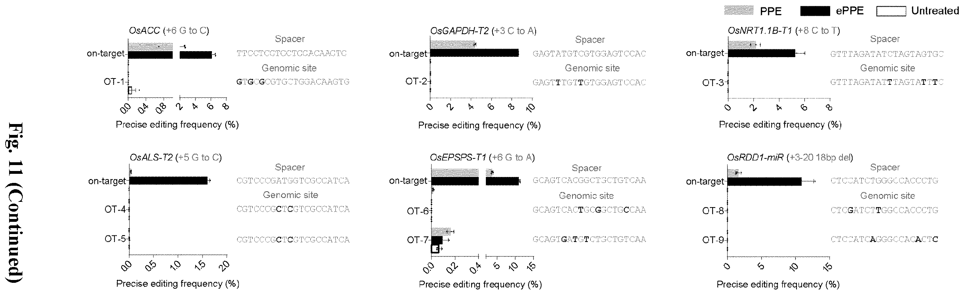

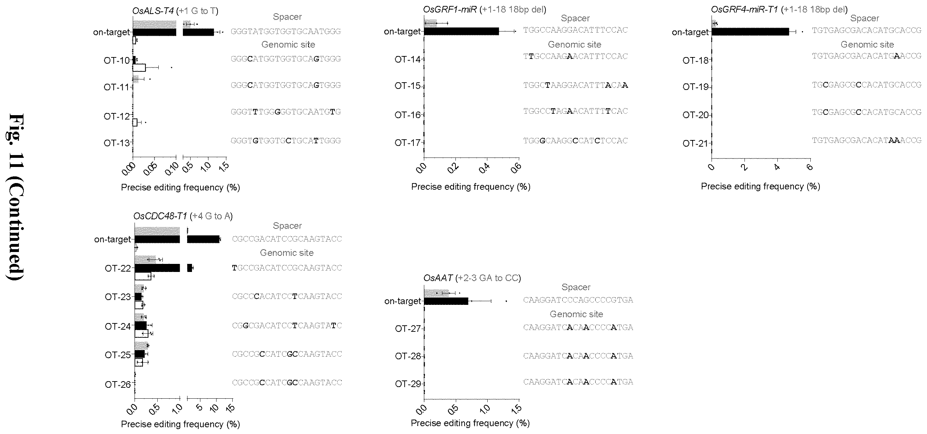

In order to determine whether ePPE significantly increased the efficiency of off-target editing, the tolerance of ePPE to the mismatch of pegRNA and the editing efficiency of potential genome off-target sites having 1-3 mismatches with the tested endogenous sites were tested respectively. The editing efficiency of the prime editing system at the endogenous targets and potential off-target sites was analyzed by amplicon sequencing analysis. The results were shown in Fig. 11. ePPE did not significantly increase the off-target editing phenomenon at most sites compared to PPE.

### Example 11: Comparison between ePPE and base editing

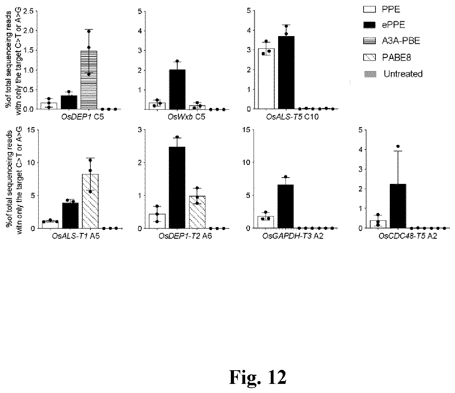

In order to compare the editing efficiency of ePPE with a commonly used base editor in aspect of introducing base mutation, 7 rice endogenous targets were selected to compare ePPE with A3A-PBE and PABE8e respectively. The results of amplicon sequencing analysis were shown in Fig. 12. Due to limitations of PAM, when C or A at other editing sites was not included, the prime editing showed a strong benefit in accurately editing target sites, ePPE and base editing were complementary in function, resulting in a more flexible application in plant genome editing.

### Example 12: Further improvement of prime editing efficiency by combining ePPE with pegRNA optimization strategy

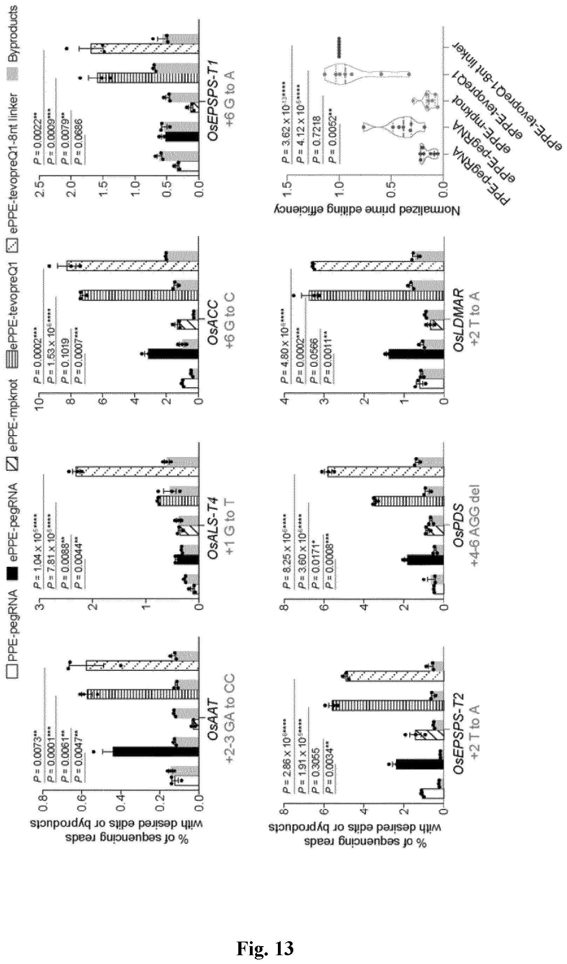

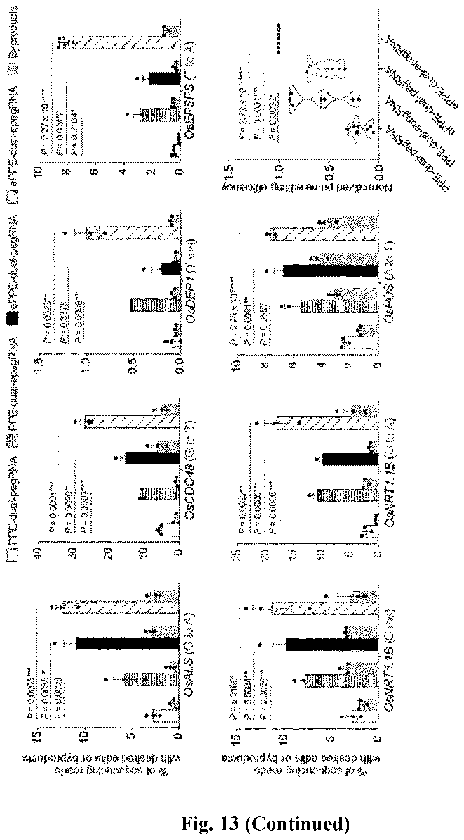

In order to further improve the plant prime editing efficiency, an ePPE protein was combined with a dual-pegRNA strategy and epegRNAs strategy. The results were shown in Fig. 13. By evaluation on different epegRNAs, it was determined to co-transform a protoplast with dual-pegRNA having a 3'-tevopreQ1-8nt linker modification with PPE and ePPE. The prime editing efficiency of the combination of the ePPE with the dual-pegRNA strategy and epegRNAs strategy was further improved, and the combination of ePPE-dual-epegRNA was 7.9 times higher than that of PPE-dual-pegRNA.

### Sequence List

>Wild-type SpCas9 amino acid sequence (SEQ ID NO: 1)
>nCas9(H840A) amino acid sequence (SEQ ID NO:2) 
MDKKYSIGLDIGTNSVGWAVITDEYKVPSKKFKVLGNTDRHSIKKNLIGALLFDSGETAEA
TRLKRTARRRYTRRKNRICYLQEIFSNEMAKVDDSFFHRLEESFLVEEDKKHERHPIFGNIV
DEVAYHEKYPTIYHLRKKLVDSTDKADLRLIYLALAHMIKFRGHFLIEGDLNPDNSDVDK
LFIQLVQTYNQLFEENPINASGVDAKAILSARLSKSRRLENLIAQLPGEKKNGLFGNLIALS
LGLTPNFKSNFDLAEDAKLQLSKDTYDDDLDNLLAQIGDQYADLFLAAKNLSDAILLSDIL
RVNTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQEDF
YPFLKDNREKIEKILTFRIPYYVGPLARGNSRFAWMTRKSEETITPWNFEEVVDKGASAQS
FIERMTNFDKNLPNEKVLPKHSLLYEYFTVYNELTKVKYVTEGMRKPAFLSGEQKKAIVD
 
LLFKTNRKVTVKQLKEDYFKKIECFDSVEISGVEDRFNASLGTYHDLLKIIKDKDFLDNEE
NEDILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAGSPAI
KKGILQTVKVVDELVKVMGRHKPENIVIEMARENQTTQKGQKNSRERMKRIEEGIKELGS
QILKEHPVENTQLQNEKLYLYYLQNGRDMYVDQELDINRLSDYDVDAIVPQSFLKDDSID
NKVLTRSDKNRGKSDNVPSEEVVKKMKNYWRQLLNAKLITQRKFDNLTKAERGGLSEL
DKAGFIKRQLVETRQITKHVAQILDSRMNTKYDENDKLIREVKVITLKSKLVSDFRKDFQF
YKVREINNYHHAHDAYLNAVVGTALIKKYPKLESEFVYGDYKVYDVRKMIAKSEQEIGK
ATAKYFFYSNIMNFFKTEITLANGEIRKRPLIETNGETGEIVWDKGRDFATVRKVLSMPQV
NIVKKTEVQTGGFSKESILPKRNSDKLIARKKDWDPKKYGGFDSPTVAYSVLVVAKVEKG
KSKKLKSVKELLGITIMERSSFEKNPIDFLEAKGYKEVKKDLIIKLPKYSLFELENGRKRML
ASAGELQKGNELALPSKYVNFLYLASHYEKLKGSPEDNEQKQLFVEQHKHYLDEIIEQISE
FSKRVILADANLDKVLSAYNKHRDKPIREQAENIIHLFTLTNLGAPAAFKYFDTTIDRKRYT
STKEVLDATLIHQSITGLYETRIDLSQLGGD
>Wild-type M-MLV-RT amino acid sequence (SEQ ID NO.3) 
TLNIEDEYRLHETSKEPDVSLGSTWLSDFPQAWAETGGMGLAVRQAPLIIPLKATSTPVSIK
QYPMSQEARLGIKPHIQRLLDQGILVPCQSPWNTPLLPVKKPGTNDYRPVQDLREVNKRV
EDIHPTVPNPYNLLSGLPPSHQWYTVLDLKDAFFCLRLHPTSQPLFAFEWRDPEMGISGQL
TWTRLPQGFKNSPTLFNEALHRDLADFRIQHPDLILLQYVDDLLLAATSELDCQQGTRAL
LQTLGNLGYRASAKKAQICQKQVKYLGYLLKEGQRWLTEARKETVMGQPTPKTPRQLR
EFLGKAGFCRLFIPGFAEMAAPLYPLTKPGTLFNWGPDQQKAYQEIKQALLTAPALGLPDL
TKPFELFVDEKQGYAKGVLTQKLGPWRRPVAYLSKKLDPVAAGWPPCLRMVAAIAVLTK
DAGKLTMGQPLVILAPHAVEALVKQPPDRWLSNARMTHYQALLLDTDRVQFGPVVALNP
ATLLPLPEEGLQHNCLDILAEAHGTRPDLTDQPLPDADHTWYTDGSSLLQEGQRKAGAAV
TTETEVIWAKALPAGTSAQRAELIALTQALKMAEGKKLNVYTDSRYAFATAHIHGEIYRRR
GWLTSEGKEIKNKDEILALLKALFLPKRLSIIHCPGHQKGHSAEARGNRMADQAARKAAI
TETPDTSTLLIENSSP
>M-MLV-RT-connection (SEQ ID NO.4) 
DQQKAYQEIKQALLTAPALGLPDLTKPFELFVDEKQGYAKGVLTQKLGPWRRPVAYLSKK
LDPVAAGWPPCLRMVAAIAVLTKDAGKLTMGQPLVILAPHAVEALVKQPPDRWLSNARM
THYQALLLDT
>RT-RNase H (SEQ ID NO.5) 
PDADHTWYTDGSSLLQEGQRKAGAAVTTETEVIWAKALPAGTSAQRAELIALTQALKMA
EGKKLNVYTDSRYAFATAHIHGEIYRRRGWLTSEGKEIKNKDEILALLKALFLPKRLSIIHC
PGHQKGHSAEARGNRMADQAARKAAITETPDTSTLL
>NC (SEQ ID NO.6)
   ATVVSGQKQDRQGGERRRSQLDRDQCAYCKEKGHWAKDCPKKPRGPRGPRPQTSLL
>PR (SEQ ID NO.7) 
TLDDQGGQGQEPPPEPRITLKVGGQPVTFLVDTGAQHSVLTQNPGPLSDKSAWVQGATGG
KRYRWTTDRKVHLATGKVTHSFLHVPDCPYPLLGRDLLTKLKAQIHFEGSGAQVMGPMG
QPLQVL
>IN (SEQ ID NO.8) 
ENSSPYTSEHFHYTVTDIKDLTKLGAIYDKTKKYWVYQGKPVMPDQFTFELLDFLHQLTH
LSFSKMKALLERSHSPYYMLNRDRTLKNITETCKACAQVNASKSAVKQGTRVRGHRPGT
HWEIDFTEIKPGLYGYKYLLVFIDTFSGWIEAFPTKKETAKVVTKKLLEEIFPRFGMPQVLG
TDNGPAFVSKVSQTVADLLGIDWKLHCAYRPQSSGQVERMNRTIKETLTKLTLATGSRDW
VLLLPLALYRARNTPGPHGLTPYEILYGAPPPLVNFPDPDMTRVTNSPSLQAHLQALYLVQ
HEVWRPLAAAYQEQLDRPVVPHPYRVGDTVWVRRHQTKNLEPRWKGPYTVLLTTPTAL
KVDGIAAWIHAAHVKAADPGGGPSSRLTWRVQRSQNPLKIRLTREAP
> M-MLV-RT-F155Y (SEQ ID NO.9) 
TLNIEDEYRLHETSKEPDVSLGSTWLSDFPQAWAETGGMGLAVRQAPLIIPLKATSTPVSIK
QYPMSQEARLGIKPHIQRLLDQGILVPCQSPWNTPLLPVKKPGTNDYRPVQDLREVNKRV
EDIHPTVPNPYNLLSGLPPSHQWYTVLDLKDAYFCLRLHPTSQPLFAFEWRDPEMGISGQL
TWTRLPQGFKNSPTLFNEALHRDLADFRIQHPDLILLQYVDDLLLAATSELDCQQGTRAL
LQTLGNLGYRASAKKAQICQKQVKYLGYLLKEGQRWLTEARKETVMGQPTPKTPRQLR
EFLGKAGFCRLFIPGFAEMAAPLYPLTKPGTLFNWGPDQQKAYQEIKQALLTAPALGLPDL
TKPFELFVDEKQGYAKGVLTQKLGPWRRPVAYLSKKLDPVAAGWPPCLRMVAAIAVLTK
DAGKLTMGQPLVILAPHAVEALVKQPPDRWLSNARMTHYQALLLDTDRVQFGPVVALNP
ATLLPLPEEGLQHNCLDILAEAHGTRPDLTDQPLPDADHTWYTDGSSLLQEGQRKAGAAV
TTETEVIWAKALPAGTSAQRAELIALTQALKMAEGKKLNVYTDSRYAFATAHIHGEIYRRR
GWLTSEGKEIKNKDEILALLKALFLPKRLSIIHCPGHQKGHSAEARGNRMADQAARKAAI
TETPDTSTLLIENSSP
> M-MLV-RT-F155V (SEQ ID NO.10) 
TLNIEDEYRLHETSKEPDVSLGSTWLSDFPQAWAETGGMGLAVRQAPLIIPLKATSTPVSIK
QYPMSQEARLGIKPHIQRLLDQGILVPCQSPWNTPLLPVKKPGTNDYRPVQDLREVNKRV
EDIHPTVPNPYNLLSGLPPSHQWYTVLDLKDAVFCLRLHPTSQPLFAFEWRDPEMGISGQL
TWTRLPQGFKNSPTLFNEALHRDLADFRIQHPDLILLQYVDDLLLAATSELDCQQGTRAL
LQTLGNLGYRASAKKAQICQKQVKYLGYLLKEGQRWLTEARKETVMGQPTPKTPRQLR
EFLGKAGFCRLFIPGFAEMAAPLYPLTKPGTLFNWGPDQQKAYQEIKQALLTAPALGLPDL
TKPFELFVDEKQGYAKGVLTQKLGPWRRPVAYLSKKLDPVAAGWPPCLRMVAAIAVLTK
DAGKLTMGQPLVILAPHAVEALVKQPPDRWLSNARMTHYQALLLDTDRVQFGPVVALNP
ATLLPLPEEGLQHNCLDILAEAHGTRPDLTDQPLPDADHTWYTDGSSLLQEGQRKAGAAV
TTETEVIWAKALPAGTSAQRAELIALTQALKMAEGKKLNVYTDSRYAFATAHIHGEIYRRR
GWLTSEGKEIKNKDEILALLKALFLPKRLSIIHCPGHQKGHSAEARGNRMADQAARKAAI
TETPDTSTLLIENSSP
> M-MLV-RT-F156Y (SEQ ID NO.11) 
TLNIEDEYRLHETSKEPDVSLGSTWLSDFPQAWAETGGMGLAVRQAPLIIPLKATSTPVSIK
QYPMSQEARLGIKPHIQRLLDQGILVPCQSPWNTPLLPVKKPGTNDYRPVQDLREVNKRV
EDIHPTVPNPYNLLSGLPPSHQWYTVLDLKDAFYCLRLHPTSQPLFAFEWRDPEMGISGQL
TWTRLPQGFKNSPTLFNEALHRDLADFRIQHPDLILLQYVDDLLLAATSELDCQQGTRAL
LQTLGNLGYRASAKKAQICQKQVKYLGYLLKEGQRWLTEARKETVMGQPTPKTPRQLR
EFLGKAGFCRLFIPGFAEMAAPLYPLTKPGTLFNWGPDQQKAYQEIKQALLTAPALGLPDL
TKPFELFVDEKQGYAKGVLTQKLGPWRRPVAYLSKKLDPVAAGWPPCLRMVAAIAVLTK
DAGKLTMGQPLVILAPHAVEALVKQPPDRWLSNARMTHYQALLLDTDRVQFGPVVALNP
ATLLPLPEEGLQHNCLDILAEAHGTRPDLTDQPLPDADHTWYTDGSSLLQEGQRKAGAAV
TTETEVIWAKALPAGTSAQRAELIALTQALKMAEGKKLNVYTDSRYAFATAHIHGEIYRRR
GWLTSEGKEIKNKDEILALLKALFLPKRLSIIHCPGHQKGHSAEARGNRMADQAARKAAI
TETPDTSTLLIENSSP
> M-MLV-RT-D524N (SEQ ID NO.12) 
TLNIEDEYRLHETSKEPDVSLGSTWLSDFPQAWAETGGMGLAVRQAPLIIPLKATSTPVSIK
QYPMSQEARLGIKPHIQRLLDQGILVPCQSPWNTPLLPVKKPGTNDYRPVQDLREVNKRV
EDIHPTVPNPYNLLSGLPPSHQWYTVLDLKDAFFCLRLHPTSQPLFAFEWRDPEMGISGQL
TWTRLPQGFKNSPTLFNEALHRDLADFRIQHPDLILLQYVDDLLLAATSELDCQQGTRAL
LQTLGNLGYRASAKKAQICQKQVKYLGYLLKEGQRWLTEARKETVMGQPTPKTPRQLR
EFLGKAGFCRLFIPGFAEMAAPLYPLTKPGTLFNWGPDQQKAYQEIKQALLTAPALGLPDL
 
TKPFELFVDEKQGYAKGVLTQKLGPWRRPVAYLSKKLDPVAAGWPPCLRMVAAIAVLTK
DAGKLTMGQPLVILAPHAVEALVKQPPDRWLSNARMTHYQALLLDTDRVQFGPVVALNP
ATLLPLPEEGLQHNCLDILAEAHGTRPDLTDQPLPDADHTWYTNGSSLLQEGQRKAGAAV
TTETEVIWAKALPAGTSAQRAELIALTQALKMAEGKKLNVYTDSRYAFATAHIHGEIYRRR
GWLTSEGKEIKNKDEILALLKALFLPKRLSIIHCPGHQKGHSAEARGNRMADQAARKAAI
TETPDTSTLLIENSSP
> M-MLV-RT-N200C (SEQ ID NO.13) 
TLNIEDEYRLHETSKEPDVSLGSTWLSDFPQAWAETGGMGLAVRQAPLIIPLKATSTPVSIK
QYPMSQEARLGIKPHIQRLLDQGILVPCQSPWNTPLLPVKKPGTNDYRPVQDLREVNKRV
EDIHPTVPNPYNLLSGLPPSHQWYTVLDLKDAYFCLRLHPTSQPLFAFEWRDPEMGISGQL
TWTRLPQGFKNSPTLFCEALHRDLADFRIQHPDLILLQYVDDLLLAATSELDCQQGTRALL
QTLGNLGYRASAKKAQICQKQVKYLGYLLKEGQRWLTEARKETVMGQPTPKTPRQLREF
LGKAGFCRLFIPGFAEMAAPLYPLTKPGTLFNWGPDQQKAYQEIKQALLTAPALGLPDLTK
PFELFVDEKQGYAKGVLTQKLGPWRRPVAYLSKKLDPVAAGWPPCLRMVAAIAVLTKDA
GKLTMGQPLVILAPHAVEALVKQPPDRWLSNARMTHYQALLLDTDRVQFGPVVALNPATL
LPLPEEGLQHNCLDILAEAHGTRPDLTDQPLPDADHTWYTDGSSLLQEGQRKAGAAVTTE
TEVIWAKALPAGTSAQRAELIALTQALKMAEGKKLNVYTDSRYAFATAHIHGEIYRRRGW
LTSEGKEIKNKDEILALLKALFLPKRLSIIHCPGHQKGHSAEARGNRMADQAARKAAITET
PDTSTLLIENSSP
> M-MLU-RT-ΔRNase H (SEQ ID NO.14) 
TLNIEDEYRLHETSKEPDVSLGSTWLSDFPQAWAETGGMGLAVRQAPLIIPLKATSTPVSIK
QYPMSQEARLGIKPHIQRLLDQGILVPCQSPWNTPLLPVKKPGTNDYRPVQDLREVNKRV
EDIHPTVPNPYNLLSGLPPSHQWYTVLDLKDAFFCLRLHPTSQPLFAFEWRDPEMGISGQL
TWTRLPQGFKNSPTLFNEALHRDLADFRIQHPDLILLQYVDDLLLAATSELDCQQGTRAL
LQTLGNLGYRASAKKAQICQKQVKYLGYLLKEGQRWLTEARKETVMGQPTPKTPRQLR
EFLGKAGFCRLFIPGFAEMAAPLYPLTKPGTLFNWGPDQQKAYQEIKQALLTAPALGLPDL
TKPFELFVDEKQGYAKGVLTQKLGPWRRPVAYLSKKLDPVAAGWPPCLRMVAAIAVLTK
DAGKLTMGQPLVILAPHAVEALVKQPPDRWLSNARMTHYQALLLDTDRVQFGPVVALNP
ATLLPLPEEGLQHNCLDILAEAHGTRPDLTDQPL
>M-MLU-RT-ΔRNase H-ΔConnection (SEQ ID NO.15) 
TLNIEDEYRLHETSKEPDVSLGSTWLSDFPQAWAETGGMGLAVRQAPLIIPLKATSTPVSIK
QYPMSQEARLGIKPHIQRLLDQGILVPCQSPWNTPLLPVKKPGTNDYRPVQDLREVNKRV
 
EDIHPTVPNPYNLLSGLPPSHQWYTVLDLKDAFFCLRLHPTSQPLFAFEWRDPEMGISGQL
TWTRLPQGFKNSPTLFNEALHRDLADFRIQHPDLILLQYVDDLLLAATSELDCQQGTRAL
LQTLGNLGYRASAKKAQICQKQVKYLGYLLKEGQRWLTEARKETVMGQPTPKTPRQLR
EFLGKAGFCRLFIPGFAEMAAPLYPLTKPGTLFNWGP
>Linker sequence (SEQ ID NO:16)
   SGGSSGGSSGSETPGTSESATPESSGGSSGGS
>gRNA scaffold (SEQ ID NO:17)
   guuuuagagcuagaaauagcaaguuaaaauaaggcuaguccguuaucaacuugaaaaaguggcaccgagucggugc
>PPE(SEQ ID NO:18) 
MPKKKRKVDKKYSIGLDIGTNSVGWAVITDEYKVPSKKFKVLGNTDRHSIKKNLIGALLF
DSGETAEATRLKRTARRRYTRRKNRICYLQEIFSNEMAKVDDSFFHRLEESFLVEEDKKHE
RHPIFGNIVDEVAYHEKYPTIYHLRKKLVDSTDKADLRLIYLALAHMIKFRGHFLIEGDLNP
DNSDVDKLFIQLVQTYNQLFEENPINASGVDAKAILSARLSKSRRLENLIAQLPGEKKNGL
FGNLIALSLGLTPNFKSNFDLAEDAKLQLSKDTYDDDLDNLLAQIGDQYADLFLAAKNLS
DAILLSDILRVNTEITKAPLSASMIKRYDEHHQDLTLLKALVRQQLPEKYKEIFFDQSKNGY
AGYIDGGASQEEFYKFIKPILEKMDGTEELLVKLNREDLLRKQRTFDNGSIPHQIHLGELH
AILRRQEDFYPFLKDNREKIEKILTFRIPYYVGPLARGNSRFAWMTRKSEETITPWNFEEVV
DKGASAQSFIERMTNFDKNLPNEKVLPKHSLLYEYFTVYNELTKVKYVTEGMRKPAFLSG
EQKKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGRLS
RKLINGIRDKQSGKTILDFLKSDGFANRNFMQLIHDDSLTFKEDIQKAQVSGQGDSLHEHI
ANLAGSPAIKKGILQTVKVVDELVKVMGRHKPENIVIEMARENQTTQKGQKNSRERMKRI
EEGIKELGSQILKEHPVENTQLQNEKLYLYYLQNGRDMYVDQELDINRLSDYDVDAIVPQ
SFLKDDSIDNKVLTRSDKNRGKSDNVPSEEVVKKMKNYWRQLLNAKLITQRKFDNLTKA
ERGGLSELDKAGFIKRQLVETRQITKHVAQILDSRMNTKYDENDKLIREVKVITLKSKLVS
DFRKDFQFYKVREINNYHHAHDAYLNAVVGTALIKKYPKLESEFVYGDYKVYDVRKMIA
KSEQEIGKATAKYFFYSNIMNFFKTEITLANGEIRKRPLIETNGETGEIVWDKGRDFATVRK
VLSMPQVNIVKKTEVQTGGFSKESILPKRNSDKLIARKKDWDPKKYGGFDSPTVAYSVLV
VAKVEKGKSKKLKSVKELLGITIMERSSFEKNPIDFLEAKGYKEVKKDLIIKLPKYSLFELE
NGRKRMLASAGELQKGNELALPSKYVNFLYLASHYEKLKGSPEDNEQKQLFVEQHKHY
LDEIIEQISEFSKRVILADANLDKVLSAYNKHRDKPIREQAENIIHLFTLTNLGAPAAFKYFD
TTIDRKRYTSTKEVLDATLIHQSITGLYETRIDLSQLGGDEFPKKKRKVELSGGSSGGSSGS
 
ETPGTSESATPESSGGSSGGSRPTLNIEDEYRLHETSKEPDVSLGSTWLSDFPQAWAETGG
MGLAVRQAPLIIPLKATSTPVSIKQYPMSQEARLGIKPHIQRLLDQGILVPCQSPWNTPLLP
VKKPGTNDYRPVQDLREVNKRVEDIHPTVPNPYNLLSGLPPSHQWYTVLDLKDAFFCLRL
HPTSQPLFAFEWRDPEMGISGQLTWTRLPQGFKNSPTLFNEALHRDLADFRIQHPDLILLQ
YVDDLLLAATSELDCQQGTRALLQTLGNLGYRASAKKAQICQKQVKYLGYLLKEGQRW
LTEARKETVMGQPTPKTPRQLREFLGKAGFCRLFIPGFAEMAAPLYPLTKPGTLFNWGPDQ
QKAYQEIKQALLTAPALGLPDLTKPFELFVDEKQGYAKGVLTQKLGPWRRPVAYLSKKLD
PVAAGWPPCLRMVAAIAVLTKDAGKLTMGQPLVILAPHAVEALVKQPPDRWLSNARMTH
YQALLLDTDRVQFGPVVALNPATLLPLPEEGLQHNCLDILAEAHGTRPDLTDQPLPDADHT
WYTDGSSLLQEGQRKAGAAVTTETEVIWAKALPAGTSAQRAELIALTQALKMAEGKKLN
VYTDSRYAFATAHIHGEIYRRRGWLTSEGKEIKNKDEILALLKALFLPKRLSIIHCPGHQKG
HSAEARGNRMADQAARKAAITETPDTSTLLIENSSPSGGSPKKKRKV
>ePPE(SEQ ID NO:19)

MPKKKRKVDKKYSIGLDIGTNSVGWAVITDEYKVPSKKFKVLGNTDRHSIKKNLIGALLF
DSGETAEATRLKRTARRRYTRRKNRICYLQEIFSNEMAKVDDSFFHRLEESFLVEEDKKHE
RHPIFGNIVDEVAYHEKYPTIYHLRKKLVDSTDKADLRLIYLALAHMIKFRGHFLIEGDLNP
DNSDVDKLFIQLVQTYNQLFEENPINASGVDAKAILSARLSKSRRLENLIAQLPGEKKNGL
FGNLIALSLGLTPNFKSNFDLAEDAKLQLSKDTYDDDLDNLLAQIGDQYADLFLAAKNLS
DAILLSDILRVNTEITKAPLSASMIKRYDEHHQDLTLLKALVRQQLPEKYKEIFFDQSKNGY
AGYIDGGASQEEFYKFIKPILEKMDGTEELLVKLNREDLLRKQRTFDNGSIPHQIHLGELH
AILRRQEDFYPFLKDNREKIEKILTFRIPYYVGPLARGNSRFAWMTRKSEETITPWNFEEVV
DKGASAQSFIERMTNFDKNLPNEKVLPKHSLLYEYFTVYNELTKVKYVTEGMRKPAFLSG
EQKKAIVDLLFKTNRKVTVKQLKEDYFKKIECFDSVEISGVEDRFNASLGTYHDLLKIIKD
KDFLDNEENEDILEDIVLTLTLFEDREMIEERLKTYAHLFDDKVMKQLKRRRYTGWGRLS
RKLINGIRDKQSGKTILDFLKSDGFANRNFMQLIHDDSLTFKEDIQKAQVSGQGDSLHEHI
ANLAGSPAIKKGILQTVKVVDELVKVMGRHKPENIVIEMARENQTTQKGQKNSRERMKRI
EEGIKELGSQILKEHPVENTQLQNEKLYLYYLQNGRDMYVDQELDINRLSDYDVDAIVPQ
SFLKDDSIDNKVLTRSDKNRGKSDNVPSEEVVKKMKNYWRQLLNAKLITQRKFDNLTKA
ERGGLSELDKAGFIKRQLVETRQITKHVAQILDSRMNTKYDENDKLIREVKVITLKSKLVS
DFRKDFQFYKVREINNYHHAHDAYLNAVVGTALIKKYPKLESEFVYGDYKVYDVRKMIA
KSEQEIGKATAKYFFYSNIMNFFKTEITLANGEIRKRPLIETNGETGEIVWDKGRDFATVRK
VLSMPQVNIVKKTEVQTGGFSKESILPKRNSDKLIARKKDWDPKKYGGFDSPTVAYSVLV
VAKVEKGKSKKLKSVKELLGITIMERSSFEKNPIDFLEAKGYKEVKKDLIIKLPKYSLFELE
NGRKRMLASAGELQKGNELALPSKYVNFLYLASHYEKLKGSPEDNEQKQLFVEQHKHY

LDEIIEQISEFSKRVILADANLDKVLSAYNKHRDKPIREQAENIIHLFTLTNLGAPAAFKYFD
TTIDRKRYTSTKEVLDATLIHQSITGLYETRIDLSQLGGDEFSGSETPGTSESATPESATVVS
GQKQDRQGGERRRSQLDRDQCAYCKEKGHWAKDCPKKPRGPRGPRPQTSLLPKKKRKV
ELSGGSSGGSSGSETPGTSESATPESSGGSSGGSRPTLNIEDEYRLHETSKEPDVSLGSTWLS
DFPQAWAETGGMGLAVRQAPLIIPLKATSTPVSIKQYPMSQEARLGIKPHIQRLLDQGILVP
CQSPWNTPLLPVKKPGTNDYRPVQDLREVNKRVEDIHPTVPNPYNLLSGLPPSHQWYTVL
DLKDAFFCLRLHPTSQPLFAFEWRDPEMGISGQLTWTRLPQGFKNSPTLFNEALHRDLAD
FRIQHPDLILLQYVDDLLLAATSELDCQQGTRALLQTLGNLGYRASAKKAQICQKQVKYL
GYLLKEGQRWLTEARKETVMGQPTPKTPRQLREFLGKAGFCRLFIPGFAEMAAPLYPLTK
PGTLFNWGPDQQKAYQEIKQALLTAPALGLPDLTKPFELFVDEKQGYAKGVLTQKLGPWR
RPVAYLSKKLDPVAAGWPPCLRMVAAIAVLTKDAGKLTMGQPLVILAPHAVEALVKQPPD
RWLSNARMTHYQALLLDTDRVQFGPVVALNPATLLPLPEEGLQHNCLDILAEAHGTRPDL
TDQPLSGGSPKKKRKV

## Claims

1. A prime editing system for targeted modification of a plant genome, comprising:
i) a prime editing fusion protein and/or an expression construct comprising a nucleotide sequence encoding the prime editing fusion protein, wherein the prime editing fusion protein comprises a CRISPR nickase and a reverse transcriptase; and/or
ii) at least one pegRNA and/or an expression construct comprising a nucleotide sequence encoding the at least one pegRNA,
wherein the at least one pegRNA comprises, in the direction from 5' to 3', a guide sequence, a scaffold sequence, a reverse transcription (RT) template sequence and a primer binding site (PBS) sequence,
wherein the at least one pegRNA is capable of forming a complex with the fusion protein and targeting the fusion protein to a target sequence in the genome, so that a nick is formed in the target sequence.

2. The system of claim 1, wherein the CRISPR nickase is a Cas9 nickase, e.g., comprising an amino acid sequence shown in SEQ ID NO: 2.

3. The system of claim 1 or 2, wherein the reverse transcriptase is a M-MLV reverse transcriptase or a functional variant thereof.

4. The system of any one of claims 1-3, wherein
(a) the reverse transcriptase comprises a mutation selected from any one of F155Y, F155V, F156Y, D524N, D200C, wherein the amino acid position refers to SEQ ID NO: 3;
(b) a connection sequence of the reverse transcriptase is deleted; and/or
(c) a RNase H domain of the reverse transcriptase is mutated or deleted.

5. The system of claim 4, wherein the reverse transcriptase comprises a sequence shown in any one of SEQ ID NOs: 9-15.

6. The system of any one of claims 1-5, wherein the reverse transcriptase, such as the M-MLV reverse transcriptase, or the functional variant thereof is fused at N-terminus or C-terminus with a nucleocapsid protein (NC), a protease (PR), or an integrase (IN) directly or via a linker in the fusion protein.

7. The system of claim 6, wherein the nucleocapsid protein (NC) contains an amino acid sequence shown in SEQ ID NO: 6, or the protease (PR) contains an amino acid sequence shown in SEQ ID NO: 7, or the integrase (IN) contains an amino acid sequence shown in SEQ ID NO: 8.

8. The system of claim 6 or 7, wherein the reverse transcriptase is fused at the N-terminus with the nucleocapsid protein (NC) directly or via a linker, and preferably, the functional variant of the M-MLV reverse transcriptase of which the RNase H domain is deleted is fused at the N-terminus with the nucleocapsid protein (NC) directly or via a linker.

9. The system of any one of claims 1-8, wherein the guide sequence of the pegRNA is configured to have sufficient sequence identity with the target sequence and thus is capable of binding to a complementary strand of the target sequence by base pairing so as to achieve sequence-specific targeting.

10. The system of any one of claims 1-9, wherein the scaffold sequence of the pegRNA comprises a sequence shown in SEQ ID NO: 18.

11. The system of any one of claims 1-10, wherein the primer binding sequence is configured to be complementary to at least a portion of the target sequence, and preferably the primer binding sequence is complementary to at least a portion of a 3' free single strand caused by the nick, in particular to a nucleotide sequence at the 3' end of the 3' free single strand.

12. The system of any one of claims 1-11, wherein the primer binding sequence has a Tm (melting temperature) of about 18-52 °C, preferably about 24-36 °C, more preferably about 28-32 °C, and most preferably about 30 °C.

13. The system of any one of claims 1-12, wherein the RT template sequence is configured to correspond to a sequence downstream of the nick and comprises a desired modification, and the modification comprises substitution, deletion and/or addition of one or more nucleotides.

14. The system of any one of claims 1-13, wherein the prime editing fusion protein contains an amino acid sequence shown in SEQ ID NO: 19.

15. A method of producing a genetically modified plant, comprising introducing the prime editing system of any one of claims 1-14 into at least one cell, thereby resulting in a modification in the target sequence in the genome of the at least one cell.
